# EUROPEAN PATENT SPECIFICATION

(11) **EP 4 255 312 B1**
(45) Date of publication and mention of the grant of the patent: **25.03.2026**
(21) Application number: 21904241.3
(22) Date of filing: 07.12.2021
(51) Int. Cl.: A61B 17/00, A61B 17/04, A61F 2/00, A61F 2/24

(54) **DEVICE AND SYSTEM FOR RESHAPING A HEART VALVE ANNULUS**
VORRICHTUNG UND SYSTEM ZUR UMFORMUNG EINES HERZKLAPPENANNULUS
DISPOSITIF ET SYSTÈME DE REMODELAGE D'ANNEAU DE VALVE CARDIAQUE

(30) Priority: 07.12.2020 US 202063122420 P
(43) Date of publication of application: 11.10.2023
(73) Proprietor: MVRx, Inc., San Mateo, California 94402 (US)
(72) Inventor: CHILDS, Richard T., San Mateo, California 94402 (US); RAHDERT, David A., San Mateo, California 94402 (US); THOLFSEN, David R., San Leandro, CA 94577 (US); WU, Patrick P., San Carlos, CA 94070 (US)
(74) Representative: Lee, Nicholas John
(86) International application number: PCT/US2021/062182
(87) International publication number: WO 2022/125535

(56) References cited:
- WO-A1-2019/028471
- US-A1- 2006 106 279
- US-A1- 2006 241 656
- US-A1- 2008 091 059
- US-B2- 10 628 042
- US-B2- 10 799 354
- US-B2- 10 799 354

## Description

### CROSS-REFERENCE TO RELATED APPLICATION(S)

This application claims benefit of priority under 35 U.S.C. §119(e) of U.S. Provisional Patent Application Serial No. 63/122,420, filed December 7, 2020.

### BACKGROUND OF THE INVENTION

### FIELD OF THE INVENTION

The present invention relates generally to medical device and procedures, and more particularly to devices, methods and systems for anchoring of an implant within the body and/or reshaping an organ within the body.

### BACKGROUND INFORMATION

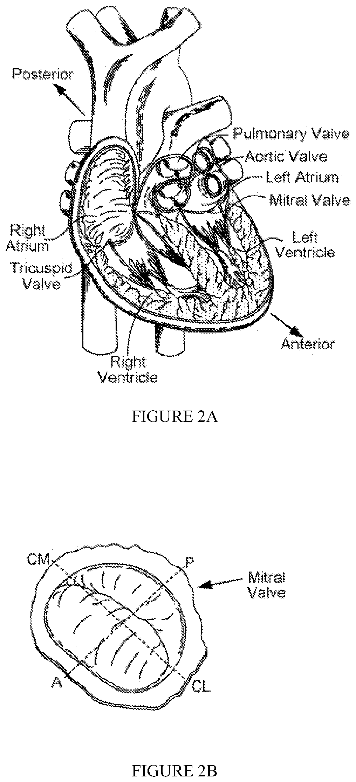

The healthy human heart (is a muscular two-side self-regulating pump slightly larger than a clenched fist, as can be seen in Figures 2A-2C. It is composed of four chambers including the right atrium (RA) and right ventricle (RV), and the left atrium (LA) and LV (LV). The RA collects poorly oxygenated blood returning from the lower body via the inferior vena cava (IVC) and from the head and upper body via the superior vena cava (SVC) and delivers it through the tricuspid valve to the RV. The RV then contracts which has the effect of closing the tricuspid valve and forcing the blood through the pulmonary valve into the pulmonary artery for circulation to the lungs. The left side of the heart collects the oxygenated blood in the LA returning from the lungs via the pulmonary veins. From, there the blood is delivered to the LV. The LV then powerfully contracts having the effect of closing the mitral valve (MV) and forcing the blood through the aortic valve into the aorta and thence throughout the body.

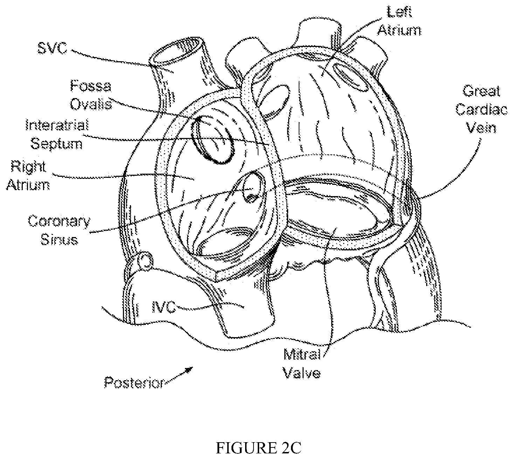

The interatrial septum, a wall composed of fibrous and muscular parts that separates the RA and LA, as can be seen in Figure 2C. The fibrous interatrial septum is, compared to the more friable muscle tissue of the heart, a more materially strong tissue structure in its own extent in the heart. An anatomic landmark on the interatrial septum is an oval, thumbprint sized depression called the oval fossa, or fossa ovalis, as can be seen in Figure 2C, which is a remnant of the oval foramen and its valve in the fetus. It is free of any vital structures such as valve structure, blood vessels and conduction pathways. Together with its inherent fibrous structure and surrounding fibrous ridge, which makes it identifiable by angiographic techniques, the fossa ovalis is the favored site for trans-septal diagnostic and therapeutic procedures from the right into the left heart. Before birth, oxygenated blood from the placenta was directed through the oval foramen into the LA, and after birth the oval foramen closes. The heart's four valves function primarily to ensure the blood does not flow in the wrong direction during the cardiac cycle e.g., backflow from the ventricles to the atria or backflow from the arteries into the corresponding ventricles.

The synchronous pumping actions of the left and right sides of the heart constitute the cardiac cycle. The cycle begins with a period of ventricular relaxation, called ventricular diastole. At the beginning of ventricular diastole (e.g., ventricular filling), the aortic and pulmonary valves are closed to prevent backflow from the arteries into the ventricles. Shortly thereafter, the tricuspid and mitral valves open to allow flow from the atria into the corresponding ventricles. Shortly after ventricular systole (e.g., ventricular contraction and emptying) begins, the tricuspid and mitral valves close to prevent backflow from the ventricles into the corresponding atria. The aortic and pulmonary valves then open to permit discharge of blood into the arteries from the corresponding ventricles. The opening and closing of the heart valves occur primarily as a result of pressure differences. For example, the opening and closing of the mitral valve occurs as a result of the pressure differences between the LA and the LV. During ventricular diastole, when the LV is relaxed, the blood returning from the lungs into the LA causes the pressure in the atrium to exceed that in the LV. As a result, the mitral valve opens, allowing blood to flow from the LA into the LV. Subsequently as the now full ventricle contracts in ventricle systole, the intraventricular pressure rises above the pressure in the atrium and pushes the mitral valve shut.

The mitral and tricuspid valves are defined by fibrous rings of collagen, each called an annulus, which forms a part of the fibrous skeleton of the heart. The annulus provides attachment to cusps or leaflets of the mitral valve (called the anterior and posterior cusps or leaflets) and the three cusps or leaflets of the tricuspid valve. The cusps of a healthy mitral valve are shown in Figure 2B. Proper closing function is also aided by a tethering action of chordae tendineae and one or more papillary muscles. Also of structural relevance to this invention and located in the vicinity of the annulus of the mitral valve is the coronary sinus and its tributaries including the great cardiac vein (GVC), as can be seen in Figure 2C. The GVC generally courses around the lower wall of the LA outside the atrial chamber but within the atrial wall. The GVC empties into the RA through the coronary sinus.

Each of the valves in question is a one-way valve that function to allow blood to flow only in the appropriate direction. If any of the valves does not function properly, that will affect the efficiency of the heart and may result in significant health issues. For example, failure of the mitral valve between the LA and the LV, to fully seal while the LV is contracting results in some portion of the blood in the LV being expelled retrograde back into the LA. This is generally termed mitral regurgitation and depending on severity, can result in insufficient blood flow throughout the body with resultant serious health implications.

### II. Characteristics and Causes of Mitral Valve Dysfunction

When the LV contracts after filling with blood from the LA, the walls of the ventricle move inward and release some of the tension from the papillary muscle and chords. The blood pushed up against the under-surface of the mitral leaflets causes them to rise toward the annulus plane of the mitral valve. As they progress toward the annulus, the leading edges of the anterior and posterior leaflet come together forming a seal and closing the valve. In the healthy heart, leaflet coaption occurs near the plane of the mitral annulus. The blood continues to be pressurized in the LV until it is ejected into the aorta. Contraction of the papillary muscles is simultaneous with the contraction of the ventricle and serves to keep healthy valve leaflets tightly shut at peak contraction pressures exerted by the ventricle.

In a healthy heart, the dimensions of the mitral valve annulus create an anatomic shape and tension such that the leaflets coapt, forming a tight junction, at peak contraction pressures. Where the leaflets coapt at the opposing medial and lateral sides of the annulus are called the leaflet commissures CM, CL, as shown Figure 2B. Valve malfunction can result from the chordae tendineae (the chords) becoming stretched, and in some cases tearing. When a chord tears, this results in a leaflet that flails. Also, a normally structured valve may not function properly because of an enlargement of or shape change in the valve annulus. This condition is referred to as a dilation of the annulus and generally results from heart muscle failure. In addition, the valve may be defective at birth or because of an acquired disease. Regardless of the cause, mitral valve dysfunction can occur when the leaflets do not coapt at peak contraction pressures. When this occurs, the coaption line of the two leaflets is not tight at ventricular systole. As a result, an undesired back flow of blood from the LV into the LA can occur.

This mitral regurgitation, if significant in amount, may have has several serious health consequences. For example, blood flowing back into the atrium may cause high atrial pressure and reduce the flow of blood into the LA from the lungs. As blood backs up into the pulmonary system, fluid leaks into the lungs and causes pulmonary edema. Another health problem resulting from mitral valve dysfunction is the reduction of ejection fraction of the heart, or the effective pumping of the blood through the body of that blood that does enter the LV. The blood volume regurgitating back into the atrium reduces the volume of blood going forward into the aorta causing low cardiac output. Excess blood in the atrium as a result of mitral valve regurgitation may also over-fill the ventricle during each cardiac cycle and causes volume overload in the LV. Over time, this may result in dilation of the LV and indeed the entire left side of the heart. This may further reduce the effective cardiac output and further worsen the mitral regurgitation problem by dilating the mitral valve annulus. Thus, once the problem of mitral valve regurgitation begins, the resultant cycle may cause heart failure to be hastened. Treating the problem therefore not only has the immediate effect of alleviating the heart output problems mentioned above, but also may interrupt the downward cycle toward heart failure.

### III. Current Treatment Methods

Various methods of treating this serious heart condition have been suggested. In one approach, the native valve is removed and replaced with a new valve, such as described in U.S. Pat. No. 6,200,341 to Jones et al and U.S. Pat. No. 7,645,568 to Stone. While this approach may be of use in some situations, such surgical procedures generally require open chest surgery, which is invasive and often contraindicated for very sick or old patients, which includes many of those suffering from mitral valve regurgitation.

Another method which has been suggested is to apply tension across the LV to reshape the LV, thereby affect the functioning of the mitral valve, such as described in U.S. 2005/0075723 to Schroeder et al. This approach uses a splint that spans across a ventricle and extends between epicardial pads that engage outside surfaces of the heart. This approach is also invasive and potentially problematic as it penetrates an outer surface of the heart.

Another method that has been suggested is the attempted constriction of the LA by means of a belt like constricting device extending inside the GVC which runs along the posterior wall of the LA, such as described in U.S. 2002/0183841 A1 to Cohn et al. While this may be partially helpful, often the device fails to sufficiently alter the shape of the left atrium to fully resolve the failure of the leaflets to coapt.

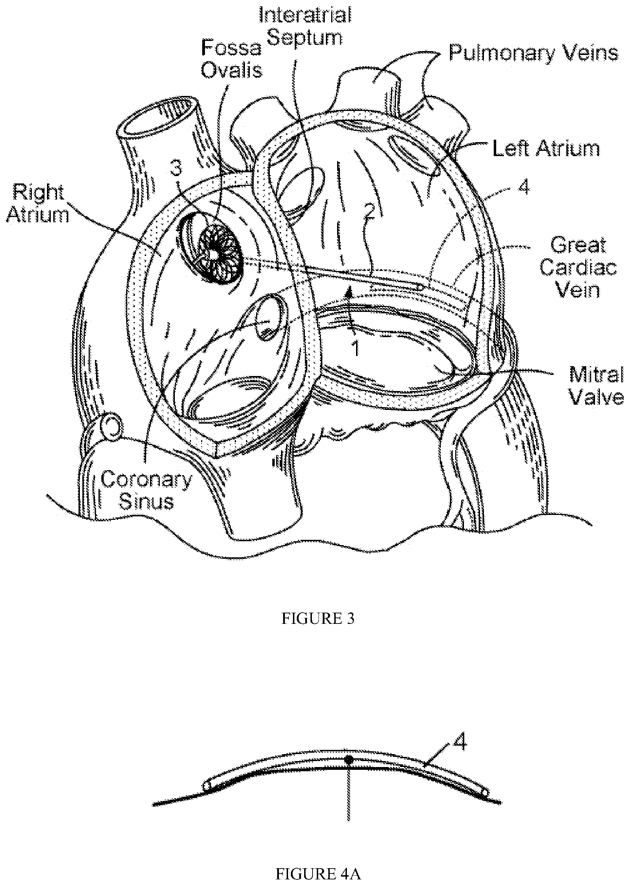

Yet another method that has proven particularly useful is to employ a system that applies direct tension across the width of the LA and across the minor axis of the annulus of the mitral valve, such as shown in Figure 3. System 1 utilizes a bridging element 2 that extends between an anterior anchor 3 and a posterior anchor 4. The anterior anchor 3 is generally located at the wall between the LA and the RA, for example, on the fossa ovalis on the septal wall, and is attached to the bridging element 2 that spans the LA. Posterior anchor 4 is located across the atrium posterior to the anterior anchor and may be located outside the atrium chamber in the GVC. The bridging element is affixed to the posterior anchor and provides a bridge across the LA between the septum. The GVC and is tensioned to directly affect the shape of the LA, and in particular, the annulus of the mitral valve. By adjusting the tension of the bringing element, the shape of the LA and particularly the annulus of the mitral valve can be adjusted to achieve optimum closure of the mitral valve during cardiac function. An example of this approach is described in detail in U.S. Pat. No. 8,979,925 B2 to Chang et al.

This approach has many advantages over conventional approaches, including avoiding invasive procedures such as open heart surgery or being placed on a heart-lung machine. However, there are still a number of challenges that must be addressed. While the anterior anchor provides relatively robust and secure anchoring with the fossa ovalis, anchoring within a body vessel, such as the GCV is more problematic. While the fossa ovalis is defined by a notable depression, which lends itself to having an anchor disposed within, the GCV lacks any notable anatomical features and is defined by a relatively smooth-walled vessel along the outer wall of the left atrium. In addition, the heart is a highly dynamic organ such that any implant disposed therein is subjected to highly variable forces and movements due to the contortions of the heart muscle during a pumping cycle of the heart. These aspects make anchoring within the GCV particularly challenging. Thus, there is need for devices, systems and methods that allow for robust and dependable anchoring within a vessel, such as the GCV. There is further need for such anchoring devices that can withstand considerable forces over the lifetime of the device. There is further need for such anchoring devices that can assist in reshaping of an organ, such as the heart.

US10799354B2 discloses an anchor system for reshaping a heart valve annulus for treatment of a heart disorder, comprising an augmentation device with a cylindrical body, wherein the cylindrical body has a cylindrical wall with one opening.

### SUMMARY OF THE INVENTION

The present invention provides systems and associated devices for delivery and deployment of heart implants for reshaping a heart valve annulus for treatment of a heart disorder, such as mitral valve regurgitation. The invention is directed to an anchor system as defined in independent claim 1.

Accordingly, in one embodiment, the invention provides an anchor system including an augmentation device and an anchor. In some aspects, the augmentation device has an elongated cylindrical body defined by a substantially cylindrical wall. The lumen is configured to receive an anchor and the cylindrical wall includes slots disposed along a length of the cylindrical body for engaging a bridging element of the anchor. The system further includes an anchor having a substantially cylindrical body that is sized to pass within the elongated cylindrical body of the augmentation device, and a bridging element coupled to an intermediate portion of the anchor.

In another aspect, the augmentation device has an elongated shaft body, wherein the shaft body has a first elongated configuration and a second flexed configuration. The second flexed configuration has a reduced length as compared to the first elongated configuration. The system further includes an anchor having a substantially cylindrical body having a length less than the that of the augmentation device, and a bridging element coupled to an intermediate portion of the anchor. The system is configured such that when the augmentation device and anchor are coupled and deployed in a body lumen, a force upon a wall of the body lumen from the anchor is translated to the augmentation device to deform the wall.

In various embodiments, an anchor system includes: an anterior anchor and a posterior anchor. In some aspects, the anchor system includes an anterior anchor having an anchor portion operable to secure the anterior anchor in tissue, a through hole extending through the anchor member, and an elongated tube having a lumen coextensive with the through hole, wherein the elongated tube is composed of a semi-rigid or rigid material that resists flexing; and a posterior anchor coupled to a first end of a bridging element, wherein a second end of the bridging element is configured to traverse the lumen of the elongated tube of the anterior anchor.

In another aspect, the anchor system includes: an anterior anchor having an anchor portion operable to secure the anterior anchor in tissue, a through hole extending through the anchor member, and an adjustable arm extending from the anchor portion; and a posterior anchor coupled to a first end of a bridging element, wherein a second end of the bridging element is configured to traverse the through hole of the anterior anchor, and wherein the adjustable arm is operable to adjust positioning of the bridging element when the anterior anchor and the posterior anchor are coupled via the bridging element upon deployment in a body vessel.

In yet another embodiment, an anchor system includes an anterior implant and a posterior anchor. In some aspects, the anterior implant has a first anterior anchor, a second anterior anchor, a connecting rail extending between the first and second anterior anchors, and a bridging element connector disposed on the connecting rail. The system further includes a posterior anchor coupled to a first end of a bridging element, wherein a second end of the bridging element is configured to engage the bridging element connector and traverse a through hole of the first anterior anchor or a through hole of the second anterior anchor when the anterior implant and the posterior anchor are coupled via the bridging element upon deployment in a body vessel. In some aspects, the bridging element connector is configured as a slidable lock slidably disposed on the connecting rail to allow adjustment of the bridging element positioning along the connecting rail.

In another embodiment, the disclosure provides a method of reshaping a heart chamber in a subject. The method includes implanting the anchor system of the invention in the heart chamber, thereby reshaping the heart chamber of the subject.

In still another embodiment, the disclosure provides a method of treating mitral valve regurgitation in a subject by reshaping a left atrial heart chamber of a subject. The method includes implanting the anchor system in the left atrial heart chamber, thereby treating mitral valve regurgitation in the subject.

### BRIEF DESCRIPTION OF THE FIGURES

Figure 1A illustrate a heart implant system that includes an inter-atrial bridging element that spans the mitral valve annulus between an anterior anchor disposed in the fossa ovalis and a posterior anchor positioned in the GVC.
Figure 1B illustrate a heart implant system that includes an inter-atrial bridging element that spans the mitral valve annulus between an anterior anchor disposed in the fossa ovalis and a posterior anchor positioned in the GVC.
Figure 2A is an anatomic superior view of a section of the human heart showing the tricuspid valve in the right atrium, the mitral valve in the LA, and the aortic valve in between, with the tricuspid and mitral valves open and the aortic and pulmonary valves closed during ventricular diastole (ventricular filling) of the cardiac cycle.
Figure 2B illustrates a healthy mitral valve demonstrating full coaptation between leaflets along the entire major axis of the valve.
Figure 2C is an anatomic anterior perspective view of the left and right atriums, with portions broken away and in section to show the interior of the heart chambers and associated structures, such as the fossa ovalis, coronary sinus, and the GVC.
Figure 3 shows a conventional implant system having a bridge spanning the left atrium between an anterior anchor disposed in the fossa ovalis and a curved posterior anchor disposed in the GCV.
Figures 4A illustrates the tendency of a conventional curved posterior anchor to flip or invert when tension forces are applied.
Figures 4B illustrates the tendency of a conventional curved posterior anchor to flip or invert when tension forces are applied.
Figure 5 illustrates a posterior anchor with a jacket attached to a tensioning member in accordance with some embodiments.
Figure 6 illustrates a posterior anchor with a jacket attached to a tensioning member, in accordance with some embodiments.
Figure 7A illustrates a posterior anchor attached to a tensioning member with an anti-flipping feature, in accordance with some embodiments.
Figure 7B illustrates a posterior anchor attached to a tensioning member with an anti-flipping feature, in accordance with some embodiments.
Figure 8 illustrates a posterior anchor attached to a tensioning member with another anti-flipping feature, in accordance with some embodiments.
Figure 9A illustrates a posterior anchor that includes a support element disposed in a far side of a compressible cylinder so as to deform the cylinder when tensioned, in accordance with some embodiments.
Figure 9B illustrates the posterior anchor in Figure 9A disposed within the GCV before and after deformation, respectively, in accordance with some embodiments.
Figure 9C illustrates the posterior anchor in Figure 9A disposed within the GCV before and after deformation, respectively, in accordance with some embodiments.
Figure 10A illustrates a heart implant system having an anterior anchor and multiple bridge elements, each extending to a separate posterior anchor within the GCV, in accordance with some embodiments.
Figure 10B illustrates a heart implant system having an anterior anchor and multiple bridge elements extending to a single posterior anchor within the GCV, in accordance with some embodiments.
Figure 10C illustrates a heart implant system for reshaping the tricuspid valve, the system having two bridge elements extending from anchors in the superior and inferior vena cava to a posterior anchor disposed in the right ventricle, in accordance with some embodiments.
Figure 11A illustrates a posterior anchor that is curvable or conformable upon adjustment of the tensioning member by use of one or more tethers, in accordance with some embodiments.
Figure 11B illustrates a posterior anchor that is curvable or conformable upon adjustment of the tensioning member by use of one or more tethers, in accordance with some embodiments.
Figure 11C illustrates a posterior anchor that is curvable or conformable upon adjustment of the tensioning member by use of one or more tethers, in accordance with some embodiments.
Figure 11D illustrates a posterior anchor that is curvable or conformable upon adjustment of the tensioning member by use of one or more tethers, in accordance with some embodiments.
Figure 11E illustrates a posterior anchor that is curvable or conformable upon adjustment of the tensioning member by use of one or more tethers, in accordance with some embodiments.
Figure 11F illustrates a posterior anchor that is curvable or conformable upon adjustment of the tensioning member by use of one or more tethers, in accordance with some embodiments.
Figure 11G illustrates a posterior anchor that is curvable or conformable upon adjustment of the tensioning member by use of one or more tethers, in accordance with some embodiments.
Figure 12A illustrates a posterior anchor defined by an expandable structure that is laterally collapsible upon tensioning of a support backbone, in accordance with some embodiments.
Figure 12B illustrates a posterior anchor defined by an expandable structure that is laterally collapsible upon tensioning of a support backbone, in accordance with some embodiments.
Figure 12C illustrates a posterior anchor defined by an expandable structure that is laterally collapsible upon tensioning of a support backbone, in accordance with some embodiments.
Figure 13A illustrates an alternative posterior anchor defined by an expandable structure having folding zones that facilitate lateral collapse upon tensioning of a support backbone, in accordance with some embodiments.
Figure 13B illustrates an alternative posterior anchor defined by an expandable structure having folding zones that facilitate lateral collapse upon tensioning of a support backbone, in accordance with some embodiments.
Figure 14 illustrates an anchor system of the present invention defined by an augmentation device having slots to allow engagement with the bridging element of a posterior anchor of the present invention.
Figure 15 illustrates an anchor system of the present invention defined by an augmentation device configured to change shape upon deployment and operable to couple to a posterior anchor in accordance with some embodiments
Figure 16 illustrates an anchor system of the present invention which includes an anterior anchor having a hypotube, in accordance with some embodiments.
Figure 17 illustrates an anterior anchor in accordance with some embodiments.
Figure 18 illustrates implantation of an anchor system in accordance with some embodiments.
Figure 19 illustrates an anterior anchor in accordance with some embodiments.
Figure 20 illustrates operation of the anterior anchor depicted in Figure 19, in accordance with some embodiments.
Figure 21 illustrates portions of the anterior anchor depicted in Figure 19, in accordance with some embodiments.
Figure 22 illustrates an anchor system which includes an anterior implant and a posterior anchor in accordance with some embodiments.
Figure 23 illustrates aspects of the anchor system depicted in Figure 22, in accordance with some embodiments.
Figure 24 illustrates aspects of the anchor system depicted in Figure 22, in accordance with some embodiments.
Figure 25 illustrates aspects of the anchor system depicted in Figure 22, in accordance with some embodiments.
Figure 26 illustrates aspects of the anchor system depicted in Figure 22, in accordance with some embodiments.
Figure 27 illustrates aspects of the anchor system depicted in Figure 22, in accordance with some embodiments.
Figure 28 illustrates aspects of the anchor system depicted in Figure 22, in accordance with some embodiments.
Figure 29 illustrates an anchor system which includes an anterior anchor and a posterior anchor in accordance with some embodiments.
Figure 30 illustrates aspects of the anchor system depicted in Figure 29, in accordance with some embodiments.
Figure 31 illustrates aspects of the anchor system depicted in Figure 29, in accordance with some embodiments.

### DETAILED DESCRIPTION OF THE INVENTION

The present invention relates to devices, systems, and methods for intravascular anchoring of an implant within the body and/or reshaping an organ within the body by use of an anchor deployed within a body lumen or body vessel. Implants described herein and associated anchors are directed to improving the function of a heart valve by reshaping a mitral valve annulus for treatment of mitral valve regurgitation. It is appreciated that any heart implant system can utilize a posterior anchor having any of the features described herein, or any combination thereof. Further, although the following embodiments describe posterior anchors for use in heart implant systems having a bridging element that spans the left atrium between an anterior anchor and the posterior anchor disposed in the GCV, it is appreciated that the features described herein pertain to implant systems for treatment of any heart valve, or can pertain to any anchor for deployment in a body lumen and could be utilized in various other implant systems at other bodily locations in accordance with the concepts described herein.

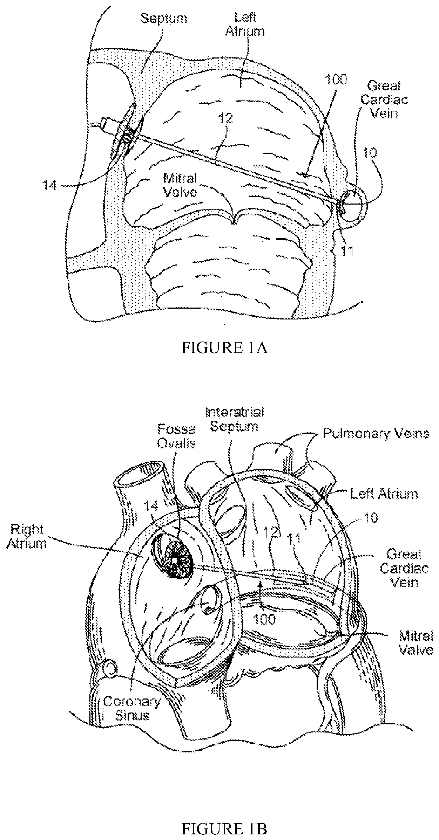

One important feature of the heart valve treatment systems for treatment of mitral valve regurgitation presented herein is the posterior anchor. As shown in the implant system 100 in Figures 1A-1B, once installed, the posterior anchor 10 is generally located within the GVC. It is important for the posterior anchor to spread tensioning forces from the bridging element as broadly as possible along the length of the GVC to avoid tearing the GVC/LA wall or pulling the posterior anchor through the tissue of the GVC/LA wall and thus reducing or eliminating the tension on the bridging element. It is also helpful to the treatment of restoring the shape and anatomical distance of the LA from the septum and the annulus of the mitral valve that the tensioning on the bridging element pull much of the LV wall in the area of the annulus forward toward the septum. If the tension is instead concentrated at a point on the LA wall, this may tend to pull just a limited point area forward and not significantly move the entire wall of the LA. The tissue may pucker or fold inward rather than pull the full wall of the LA forward.

Unlike previous GCV device concepts where the device is placed solely within the GCV to reshape the left atrium, these systems rely on additional lateral force applied to the LA wall that is supplied by, attached to and maintained by an anchor on the substantially thicker and robust septal wall to a preferred septal-lateral spacing that is controlled by the operator. Although GCV only devices attempt to reshape the path of the GCV inward, their ability to move surrounding tissue, including portions of the ventricle, is severely limited all applied forces must resolve or balance in the GCV itself. There is a need for an anchor for the GCV that distributes these substantially large forces in a manner that uniformly moves the lateral wall to cause the leaflets to co-apt without trauma or erosion, ideally maintaining as much of the natural shape, contour, and function of the GCV and the septal-lateral spacing with the septum as possible.

Among the challenges associated with such implant systems is the difficulty in providing stable, secure engagement of the posterior anchor along the posterior wall of the left atrium while disposed within the GCV. First, since the inside wall of the GCV along the left atrium is generally smooth-walled without any notable anatomical features, the posterior anchor has a tendency to slide or move, which can lead to variability of the septal-lateral spacing provided by the implant system such that some level of mitral valve regurgitation may still occur. Furthermore, since the heart is subjected to a significant amount of cyclical movement during the cardiac cycle, this sliding movement of the posterior anchor over time can lead to erosion of tissues or enlargement of the penetration through which the bridging element extends, leading to tearing of the LA wall along the GCV. Secondly, in such systems having curved or flexible posterior anchors, the curvature of the anchor often does not match the natural curvature of the atrium wall such that the posterior anchor fails to consistently engage a large enough portion of the posterior wall of the left atrium to ensure a desired reshaping of the annulus is maintained throughout the entire cardiac cycle. To address these challenges, presented herein are anchors having improved design features that provide increased stability and consistency in anchoring as well as improved engagement with adjacent tissues, particularly when deployed in a body vessel. In one aspect, the anchor has an elongate main body sized and dimensioned for delivery and deployment within the vasculature of the patient. For heart implant systems, such anchors can have a length dimension between 1 cm and 10 cm, typically between 2 cm and 8 cm, so as to distribute laterally applied anchoring forces and engage a substantial portion of the heart wall. The anchor can have a width dimension of between 0.5 cm and 5 cm, typically between 1 cm and 3 cm. The anchor can be contoured or curved along its length dimension, as well as along a width dimension, so as to conform more closely to an anatomy of the body lumen or an adjacent organ. In some embodiments, the anchor is specially shaped so as to engage at least a portion of one side of the vessel in which it is deployed, while leaving the remainder of the vessel open to facilitate blood flow therethrough. Examples of such shapes includes a D or C-shape, as well as an ovoid shape, all of which increase the contact area of the posterior anchor along the one side of the body vessel, while maintaining patency of the vessel.

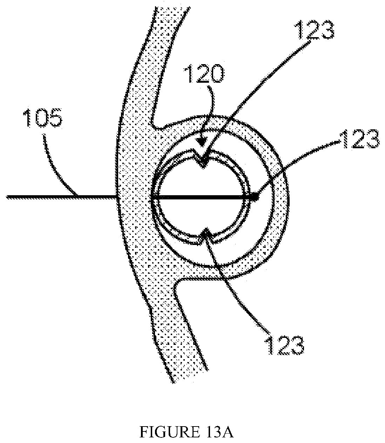

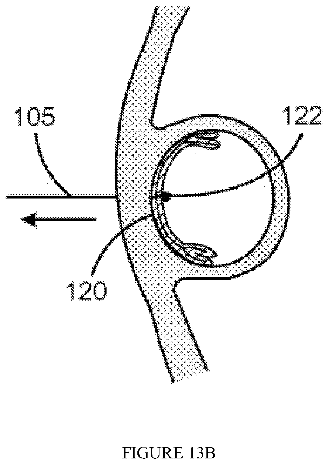

Figures 1A-1B illustrate an example heart valve treatment system 100 that includes bridging element 12 that spans across the left atrium, extending between anterior anchor 14 secured in the fossa ovalis and posterior anchor 10 deployed in the GCV. In this embodiment, posterior anchor 10 is a cylindrical structure, such as those detailed in Figure 13A, that is laterally collapsible so as to provide an increased contact surface area along the inner wall of the GCV along the wall of the LA when deployed. As can be seen in Figure 1B, posterior anchor 10 is also curved along its length so as to conform more closely with the anatomy of the outside curvature of the LA along which the GCV extends. Posterior anchor 10 can further include an anti-flipping feature 11 to inhibit flipping or inversion along its length due to movement and forces caused imparted by the structures of the heart during the cardiac cycle. While a particular design of posterior anchor is shown in Figures 1A-1B, it is appreciated that system 100 could utilize any suitable posterior anchor, including any of those described herein or any suitable anchor features in accordance with the concepts described herein.

In some embodiments, the intravascular anchors are defined as an elongate member having a central rigid portion along where the tensioning member attaches and flexible outer ends. The central rigid portion can include a stress-relief feature such as an attachment point that is flexible, movable or pivots to accommodate abrupt movements of the tensioning member so as to maintain engagement of the anchor with adjacent tissues during the heart cycle. The flexible outer ends can be provided by a modifications to the central rigid portion (e.g. notches, kerfs), or can be provided by additional components, such as a polymer jacket or cover that fits over the rigid portion.

In some embodiments, the intravascular anchor is contoured or shaped to conform to at least a portion of one side of the vessel in which it is disposed. In some embodiments, the intravascular anchor has a fixed shape, while in other embodiments, the shape of the anchor is flexible or conformable. In some embodiments, the intravascular anchor can assume multiple configurations of varying size and shape to facilitate delivery and deployment. In any of the embodiments described herein, the anchor can be defined with a hollow lumen therethrough to facilitate intravascular delivery via a guidewire or catheter.

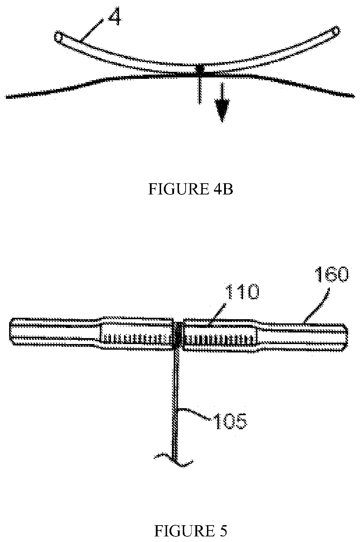

These and other aspects of the improved anchor can be further understood by referring to the embodiments depicted in Figures 5-13B. While these embodiments describe a posterior anchor for use in a tensioned heart implant, it is appreciated that these anchor features can apply to various other types of anchors for implants in various other bodily locations. For example, any of the features described can be used in an implant to provide improved anchoring, which can include improved conformance against anchored tissues, improved distribution of forces, and improved engagement of tissues to facilitate reshaping of a body organ.

Figure 5 illustrates a posterior anchor defined as a T-bar 110 that is jacketed to provide strain relief and an atraumatic tip configuration. In some embodiments, a thin or thick walled polymeric jacket 160 can be fit over a conventional rigid T-bar anchor to provide an atraumatic surface. T-bar 110 is coupled with the bridge element 105, which can be a suture, tether, or any element suitable for spanning across the left atrium and maintaining tension sufficient to reshape the atrium. The jacket 160 is sized and dimensioned so that the end portions of the jacket extend beyond the ends of the rigid T-bar 110. Jacket 160 can be formed of PTFE, high silicone soft-block urethanes, silicones, or any suitable material and can further include a thin fabric outer covering, such as polyester. In some embodiments, the jacket is preferably formed of a material that encourages tissue ingrowth. The jacket may be held in place by adhesive or shrunk over the T-bar or both. In this embodiment, jacket 160 is defined as two end pieces abutting the inner attached central bridge attachment, although the jacket could be defined a single piece jacket attached over an entire length of the T-bar, such as in the next embodiment described below. The tip extensions may be shaped to reduce tissue strain, for example curved or serpentine (not shown) to increase stability and aid delivery. This approach allows a conventional T-bar anchor to be retrofit so as to change a size and/or shape of the anchor, provide improved or variable flexibility along its length or provide various other advantageous characteristics.

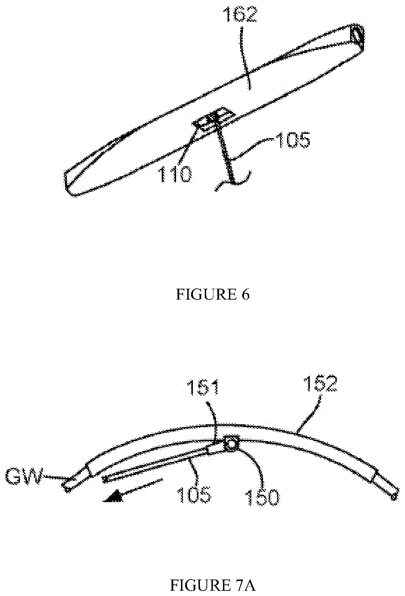

Figure 6 illustrates another posterior anchor configured as a rigid T-bar backbone 110 covered by a shaped jacket 162. Shaped jacket 162 can be polymeric semi-rigid or compliant "surfboard" that fits over the rigid T-bar 110. Such a configuration is advantageous as it allows a conventional rigid T-bar anchor to be retrofit to assume any shape, contour or flexibility desired for a particular application. In this embodiment, which is configured for use in the heart implant system described above, the shaped jacket 162 is shaped to be planar or flattened on one side so as to increase tissue contact area with the interior wall of the GVC toward the LA and to further distribute anchoring contact forces. The planar portion can be flat or curved to accommodate the shape of the vessel. In this embodiment, the planar portion is included on a center portion having increased width than either end portion and includes an opening near a center of the planar center portion, which facilitates engagement of the planar center portion with the wall of the vessel. This increased width dimension and planar portion provide improved resistance to flipping. Shaped jacket 162 can be formed thin along its posterior/anterior dimension so that it lies relatively flat against the GCV wall, thus maximizing blood flow in the GCV. This configuration also served to stabilize posterior anchor and resist flipping. As with other embodiments, surfaces may be coated or constructed of material that induces tissue ingrowth. Shaped jacket can be formed of various polymeric materials, including PTFE, high silicone soft-block urethanes, silicones, other implant grade elastomers. An optional thin fabric may be employed, such as polyester covering the polymeric jacket, to promote tissue growth or inhibit sliding. The size of the device can vary, of course depending on the desire of the surgeon and the particular requirements of the patient, for example a large male vs. a pediatric patient, but one advantageous size for typical adult patients would be, for example, 12F round or oval shaped T-bar. Such a link could be combined as a "backbone" to stabilize and strengthen other jacketed or wire form structures discussed above. The wire form may be metal, plastic, or any other material that will allow the rigid backbone to collapse the form as described above.

Although a straight version of shaped jacket 162 is shown in Figure 6, it is appreciated that shaped jacket 162 could be formed with a predetermined curved shape along its length to match the curvature of the mitral annulus or the GCV or both. Having a width close to that of the GCV, gaining more purchase of the lateral wall, the tendency of the curve to flip or right would be thwarted. In some embodiments, a delivery catheter used to deliver the anchor can include mounting features that allow axial rotation to allow proper placement of the anchor aligning the curvature with the GCV. Such feature can include lumens or guides that or any interfacing feature to allow manipulation of an orientation of the anchor during deployment. Shaped jacket can be constructed from a semi-rigid material to allow tracking over a guidewire with quasi straightening of its shape and more significant bending upon removal of the guidewire and release of the device. One or more radio-opaque features can be added to the anchor to allow a clinician to visualize its position and orientation during delivery and deployment. While in these embodiments, bridge element 105 is depicted as a suture that is wound about a mid-portion of the T-bar 110, it is appreciated that various other bridging elements and suitable means of attachment (e.g. adhesive, welding, couplings) could be used.

While some conventional systems have utilized curved posterior anchors, such anchors have a tendency to flip (when of a rigid construction) or invert (when of a more flexible construction). This action can be further understood by referring to the conventional heart valve treatment system 1 shown in Figure 3, which includes a bridging element 2 extending from an anterior anchor 3 to a mid-point of a conventional posterior anchor 4, defined as rigid curved tubular member. When a thin curved posterior anchor, especially a rigid curved anchor, is placed in the GVC, and tension is applied to the internal curvature of the arc, especially near the apex, the forces will have a tendency to flip the curved anchor in the GVC and present the exterior edge of the curvature to the passage between the GVC and the atrium.

Figures 4A-4B illustrate this flipping tendency. Flipping the anchor reaches a more stable energy condition, and therefore this is the configuration the anchor will tend to seek. In considering this flip in configuration, it is important to remember that the distal anchor, in place in the GVC, is far from a still curved structure lying against static curved vein. It is in place in a vessel full of flowing blood imbedded in the wall of a heart that is beating generally as many as 75 times or so a minute. As the posterior anchor is tossed about and buffeted by flowing blood, the anchor will quickly seek the most stable orientation in relation to the tension forces from the bridging element, and flip into the orientation with the apex of the curve pointed toward the tensioning element and the apex being pulled into the hole in the GVC/LA wall where the bridging element is pulling it unless some mechanisms, for example any of those described herein, are instituted to prevent flipping from occurring. When flipped or inverted, the anchor structure tends to focus the tensioning forces applied by the bridging element on the GVC/LA wall at a single point, the point of puncture between the LA/GVC wall. This increases the likelihood of tearing the wall and possibly pulling the posterior anchor into the atrium and releasing the tension altogether, or pulling partway into the atrium and relieving the tension to the point that the therapy is severely compromised.

This flipping movement described above would also be considerably less effective in pulling the wall of the LA toward the septum to affect reshaping of the annulus, thus would be less effective in providing therapy. With only a single point of contact between the curved posterior anchor and the GVC inner wall, the posterior anchor would be more likely to slide longitudinally within the GVC, whereupon the suture forming the bridging element would be more likely to slice the tissue forming the GVC/LA wall and expand the puncture hole, making it even more likely that the posterior anchor might get pulled through into the LA. Therefore, anti-flipping configurations and features can simultaneously provide an anti-sliding mechanism which would be doubly advantageous.

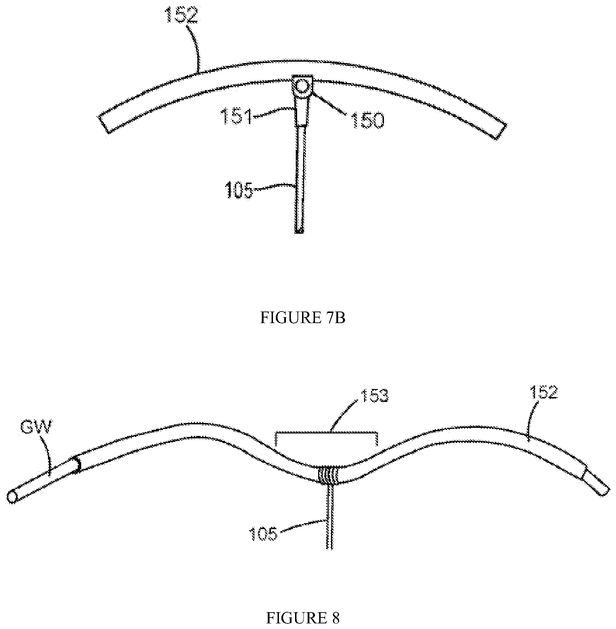

One such anti-flipping anchor configuration is shown in Figures 7A-7B. This anchor employs a rigid short link 151 that is attached by a hinge 150 or similar flexible attachment mechanism extending from the inside curve of anchor body 152. Link 151 is a relatively rigid length that can rotate to lay nearly flat against the inside curve of the curved anchor body 152 during delivery via a guidewire GW, as shown in Figure 7A, and opens to be generally perpendicular to the anchor, as shown in Figure 7B, when deployed by pulling the bridging element through a penetration in the wall of the LA. Typically, in the deployed configuration, the distal end of link 151 protrudes slightly into the LA in its resting position. In some embodiments, the link 151 is hollow such that the flexible bridging element 105 is attached to the curved posterior anchor body 152 through the hollow link 151. In other embodiments, the bridging element 105 is attached to the end extended away from anchor body 152. Link 151 is of sufficient length to cause coaxial alignment with tensioned bridging element 105 and prevent anchor from flipping over. Link 151 can be formed of a material such as plastic or smooth metal, and have a sufficient diameter that is less likely than the bare bridging element, for example a suture, to cut the tissue of the wall of the GVC where the penetration is made between the atrium and the GV. The link thus serves the double purpose of preventing flipping and protecting the wall of the GVC. The link is set to fold flat, pointing towards the puncture site during delivery and opening perpendicularly as the suture is tensioned at that site.

Figure 8 illustrates another anchor embodiment, which includes an anti-flipping or anti-flipping feature defined as an inwardly curved portion 153 along where bridging element 105 attaches to the anchor body 152. When used within a left atrium implant for treatment of MVR, the inwardly curved mid-section projects into the plane of the generally GCV shaped curved anchor with the bridge 105 attached at the midsection of the anti-flipping curved portion 153. This allows for a simpler attachment to the anchor avoiding the complications of a linking mechanism both in its construction and delivery.

In another aspect, the posterior anchor can be configured with a delivery configuration and deployed configuration in which the anchor is eccentrically disposed along one side of a vessel wall. Such configurations can include structures and materials that are expandable as well as compressible so as to form an eccentric shape, which is non-circular and having a greater surface area on one side, which is to be engaged against a wall of the body lumen or vessel. Examples of such configuration are illustrated in the following embodiments.

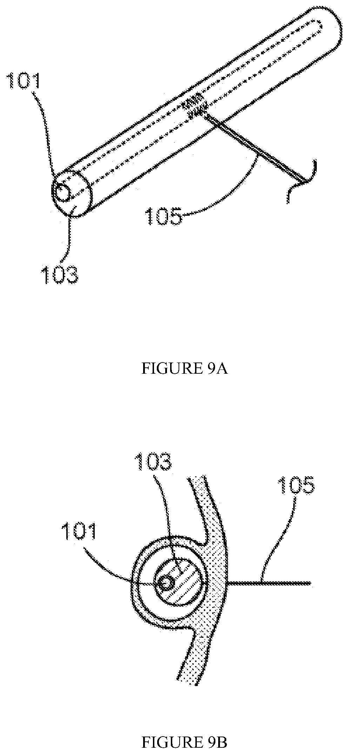

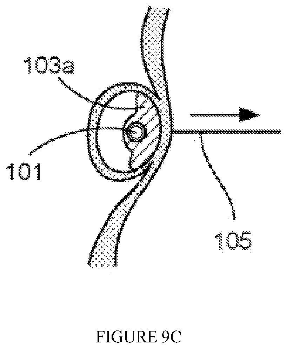

Figures 9A-9C illustrate a posterior anchor defined as crushable cylinder 103 with a more rigid support member 101, such as a T-bar support, attached or embedded within the cylinder. While a cylinder is described in this embodiment, it is appreciated that such an anchor could be configured in various elongate shapes including but not limited to partial cylinder, a crescent, an ovoid or various irregular shapes. Crushable cylinder can be formed of any suitable crushable material, such as a foam material or structure. Typically, rigid support member 101 is attached or embedded in the outer posterior diameter furthest from where the bridging element 105 extends, such as shown in Figure 9A, so as to facilitate further crushing of the cylinder when the bridging element is tensioned. The rigid support member 101 can be substantially straight, as shown, or can be curved to generally follow the curve of the interior wall of the GVC and thus spread the pulling forces uniformly against the tissue wall.

Figures 9B-9C illustrate cross-sections of the posterior anchor of Figure 9A disposed in the GVC before and after deployment, respectively. When delivered into the GVC, and connected to the bridging element 105, the crushable cylinder 103 is adjacent the wall of the GVC and LA, through which the bridging element 105 extends and the rigid support element 101 is disposed on the side furthest from the LA, as shown in Figure 9B. Upon application of tension on the bridging element to the T-bar 101, the crushable material is collapsed into an eccentric shape 103a that has a reduced cross-section which is less obstructive of blood flow within the GVC. The crushed cylinder also assumes a shape which both more closely adheres to the inner shape of the GVC, thereby increasing the contact surface area as compared to the uncrushed cylinder. When crushed, the materials also somewhat compacted and generally stiffer than the uncrushed material which also helps spreads the forces applied by the bridging element over the surface area of the GVC wall.

It is appreciated that although the embodiment shown in Figures 9A-9C are shown as a relatively short elongated crushable member and T-bar, the T-bar or spine may be significantly longer to spread the pulling force and may be shaped with a curve to spread the force more generally in the curved shaped GVC.

In some embodiments, the crushable materially is a material that encourages tissue ingrowth and or scarring to create a tissue-anchor matrix. This ingrowth further aids in assuring that the posterior anchor is not pulled through the GVC wall or flipped within the GVC. This crushable material may be constrained by the delivery catheter in a crushed form to lower its delivery profile thus aiding delivery, and when released is further reshaped to its final dimension by the bridging element.

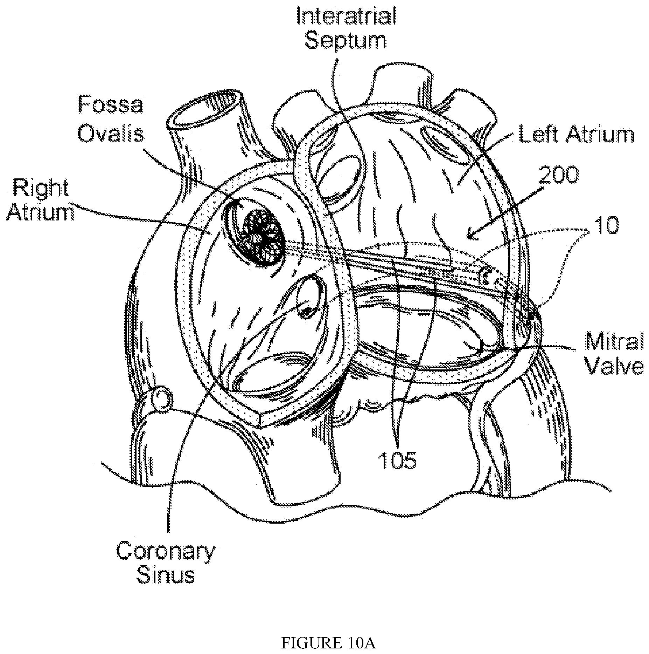

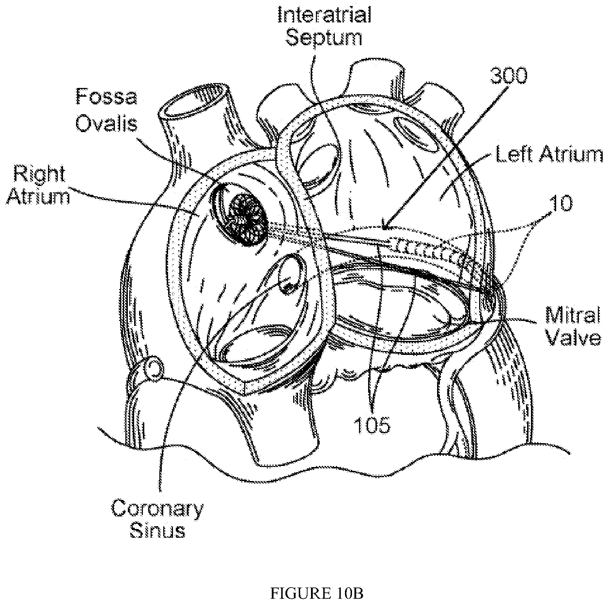

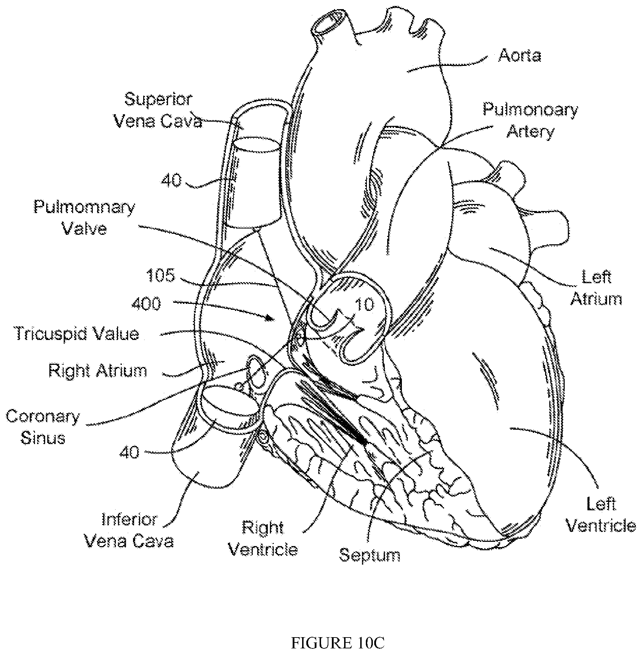

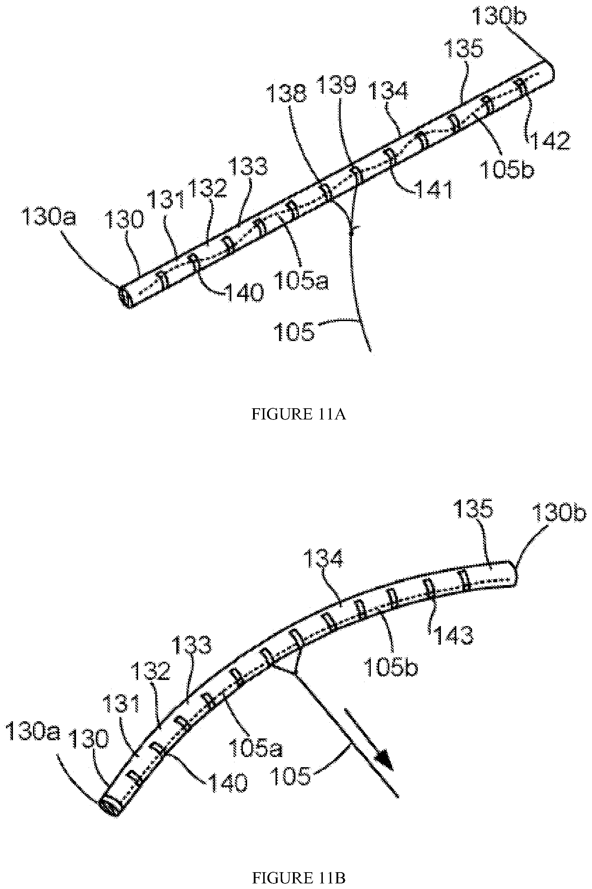

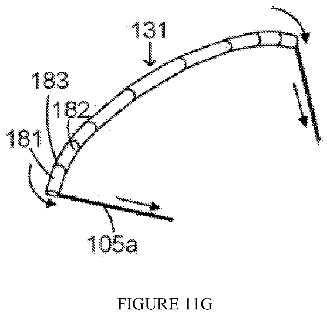

Figures 10A-10B illustrate alternative implant systems that can utilize posterior anchors in accordance with those described herein. Figure 10A illustrates a heart implant system 200 having an anterior anchor and multiple bridge elements 105 extending to multiple posterior anchors 10 within the GCV. In this embodiment, the posterior anchor 10 is a collapsible cylindrical structure, such as that described in Figure 13A. Figure 10B illustrates a heart implant system 300 having an anterior anchor and multiple bridge elements 105 extending to a single posterior anchor 10 deployed within the GCV. In this embodiment, posterior anchor 10 is a segmented tube, such as that described in Figure 11G. It is appreciated that each of the posterior anchors depicted can utilize any one or combination of the anchor features in any of the embodiments described herein. Figure 10C illustrates a heart implant system 400 for reshaping the tricuspid valve, the system having two bridge elements extending from anchors 40 in the superior and inferior vena cava to a posterior anchor 10 disposed in the right ventricle, in accordance with some embodiments. In this embodiment, the posterior anchor 10 is a collapsible cylindrical structure, such as that described in Figure 13A.

In another aspect, curved posterior anchors are provided that can be transformed from a substantially linear configuration to a curvilinear configuration. In some embodiments, the curve of the anchor can be adjusted during deployment. Some such posterior anchors include a series of interfacing or interconnecting components that articulate into a curved shape when tensioned, either by the bridging element or by one or more tethers extending therethrough. These anchors can be configured for use with systems having a single bridging element per anchor, such as that shown in Figure 10A, or in systems having multiple bridging elements, such as that shown in Figure 10B. In some embodiments, the curveable posterior anchor is defined within a single tube having a series of cuts or kerfs that allow for controlled articulation or curvature of the anchor body by the tensioned bridge. Adjustment of such anchors can include multiple schemes and anchor configurations. Examples of such configurations are detailed further below.

Figures 11A-11D illustrate a posterior anchor configured that curves inwardly toward the bridging element when deployed. Such as configuration can be designed to match a curvature of a vessel or an adjacent tissue or organ wall, and further resists flipping since the curvature can be maintained by the tensioned bridge element. Typically, the posterior anchor is defined so as to match the curvature of the GVC to more evenly and securely spread the anchor forces provided by the attachment through the bridging element which is tensioned against the anterior anchor.

The embodiments of Figure 11A-11D can be a segment tube formed from a single tube. One way this can be accomplished is to cut a hollow metal or polymeric tube 130 of a suitable length (e.g. a length that matches the mitral annulus along the GVC) into a series of segments 131,132,133 by a series of cuts called kerfs 140,141,142, as shown in Figure 11A. The kerfs can be a depth for example, of 1/2 to 3/4 of the diameter of the tube, and can also be angled to facilitate tighter radius of curvature. These areas are open, meaning that some material is cut out of the tube to define a series of segments, which allows the tube to preferentially bend in the direction of the kerfs when force is applied to both ends 130a, 130b.

One or more tethers can be used to draw segments inward to curve the anchor. In some embodiments, the internal tethers 105a, 105b are each fixed internally at the respective ends 130a, 130b of the tube and allowed to exit along a center portion of the anchor through one of the kerfs or perhaps two of the kerfs 138,139 (for example, as in Figures 11A-11B), and a bridging element is attached to the exposed tethers. Tensioning the bridging element against the GCV wall simultaneously shortens the minor axis of the mitral valve and bends the anchor to the desired shape. Such a configuration causes tube 130 to curve when the bridging element 105 is tensioned. The more tension applied, the greater the curvature toward the bridging element, until the kerf openings are closed or the engaged tissue exerts an equal countering force on the tubular body 130. This is particularly advantageous for use in a dynamic environment, such as the heart, since the aforementioned flipping typically occur when the bridging element experiences heightened tension.

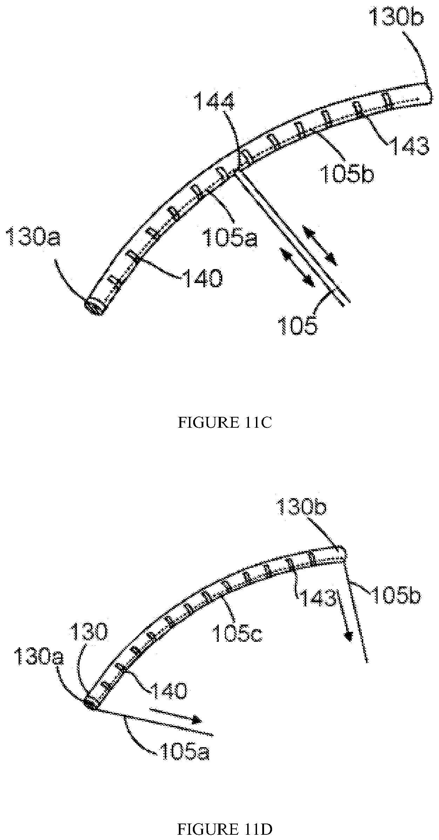

Figure 11C illustrates a similar embodiment having internal tethers 105a, 105b that are coupled with ends 130a, 130b and that exit through a central opening 144 and couple with the bridging element 105. Alternatively, tethers 105a, 105b can be each independently fixed to ends 130a, 130b and exit from the center of the anchor so as to allow for independent bending of each end. This approach can provide a configuration that provides for multiple segments and custom shaped anchor.

Figure 11D illustrates an alternative embodiment in which the bridging element 105 is a loop that extends through the tubular body 130 of the anchor such that, when tensioned by shortening the loop, the internal tether portion 105c shortens and tensioned tether portions 105a, 105b force ends 130a, 130b inward, thereby curving the anchor body. The length of the loop can be shortened by pulling one or more free ends of the loop through and attaching to the anterior anchor, thereby allowing the user to adjust the tension of the bridging elements.

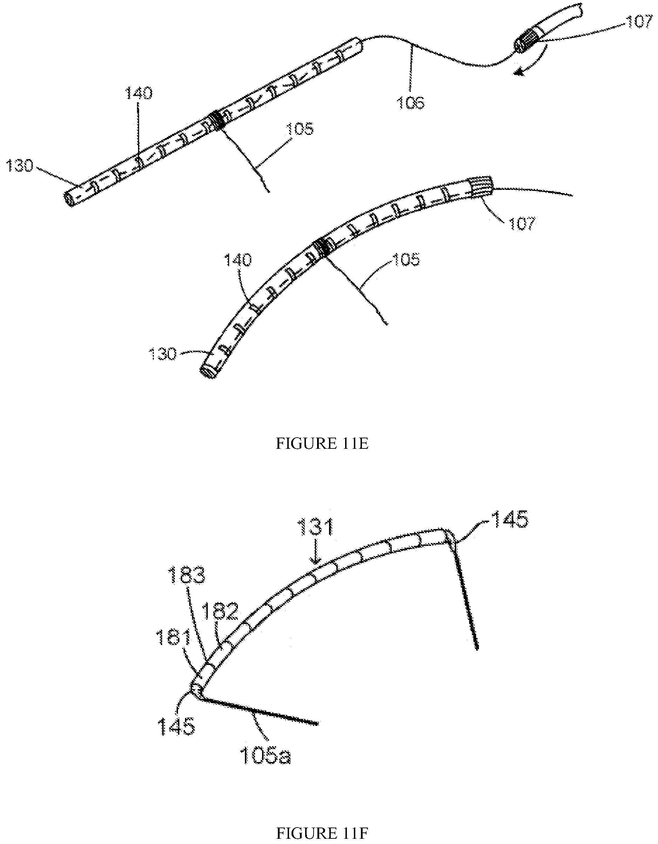

Alternatively, the bending may be independent of the bridging element. Figure 11E illustrates an example of such a bending scheme using a catheter in the GCV to pull on an internal tether 106 fixed internally to the distal end of the anchor though a lumen of the catheter. This causes the anchor's proximal end to engage the catheter tip and bend. A fastener 107, such as a clip, knot or any suitable mechanism, can be used to fix the bent anchor in the desired curved position and the excess tether is cut free.

It is appreciated that the bent configuration and the force required to bend the tube, as well as the stiffness of the bent tube can be varied as desired by adjusting the number, width, spacing and depth of the kerfs. The kerfs may be of varied length along the anchors length, combining wider and narrower sections to relatively stiffen or soften sections respectively. The curving of anchor may be achieved a single shared connected bridge or dual independent bridge elements with the latter allowing for more relaxed curve one end.

In another similar approach, the anchor is defined by individual unconnected hollow links that are similar or tailored in length. The links are formed so as to have a desired stiffness and shape for their resting location when deployed. The links can be formed using any of the constructions detailed herein. Such embodiments can utilize a delivery scheme having a single bridge with a first bridge end deployment followed by loading of the anchor or anchor links to their resting location followed by deployment of the second bridge. The tips of the anchor or outer links may have grommets or other means of protecting tissue from any abrasion from the bridging element.

In another aspect, a hybrid concept of a bendable GVC anchor with two end bridges is provided. An example of such an embodiment can include a bendable anchor resembling a string of segments or interfacing elements that extends between bridge elements and attached at each end. In some embodiments, the bridging elements are permanently fixed to each end of the anchor. The first bridge is preferably deployed farthest from the coronary sinus followed by the second with a spacing between the punctures equal to length of the anchor, which would preferably be centered over the larger central scallop leaflet of the mitral valve. The anchor is then deployed by pulling both bridges and the anchor through a protective sheath. In some embodiments, the ends of the individual segments are angled so that when the entire string is pulled tight and the ends abut, the length of the string of segments forms a curved structure. The curved structure can be preselected dependent on the angles of the segments, and need not be a constant curve. For example, such an anchor could include a relatively straight section at the center of the anchor and a more sharply curved section at each end. Alternatively, an anchor could include a straight segment and an even more sharply curved segment on the other end of the anchor, which may be a useful configuration in some applications.

Figures 11F and 11G illustrate examples of the above described alternative approach for achieving a curved posterior anchor by use of individual links. The links can be unconnected with interfacing surfaces between each, or can be interconnected in a manner that allows relative movement between adjacent links to allow for curvature of the anchor. In these depicted embodiments, tube 131 is formed by a number of individual segments 181, 182, which can be shaped with mating surfaces 183 that are either straight or angled as desired. In the embodiment of Figure 11F, the end of the anchor tube may be protected by grommets 145 connected to bridging elements 105a, 105b. In some embodiments, the grommets 145 are configured as fixed stops fixing a bridging element or tether extending therethrough to a preset length so as to provide a pre-determined curvature to the anchor. In the embodiment of Figure 11G, the links of the anchor are laced over a single bridging element or tether and are free to move along the bridge such that shortening of the bridging element or tether engages opposite ends of the anchor so as to curve the anchor. Such a configuration allows links to be added or configured to vary length or stiffness along the anchor. In either embodiment, the two bridging elements 105a, 105b may be attached to the same location on the anterior anchor. Applying tension to those bridging elements curves tube 131 inward. When such an anchor is incorporated into a heart implant system, the curved tube 131 pulls the entire wall of the LA toward the septum and advantageously shapes the mitral valve annulus with the operator able to bias the length towards toward one side or the other while viewing the regurgitant flow on ultrasound in real time. Although the links or segments are shown here as hollow tubular segments, it is appreciated that the links could be formed in various sizes and shapes, including shapes contoured to match a curvature of a vessel or the patient's anatomy. In some embodiments, the links are defined as a string of interfacing element such that shortening of the bridging element or tether articulates the links into a curved arrangement along the anchor. The interfacing elements can be of any suitable construction (e.g. solid, hollow) and can be of formed in any shape desired.

Similar to these examples, in that the configurations requires multiple bridging element attachment to the anterior anchor, would be a sequence of posterior anchors each separately attached, such as shown in Figure 10A. Such a configuration would make possible separate individual attachments that could apply tension at various angles to optimally deform the LA wall and mitral valve annulus to reduce mitral regurgitation. Each posterior anchor could employ the shapes and features of any of the posterior anchors described above. Each could attach to the same location on the anterior anchor, or could attach at slightly different locations in the anterior anchor or even separate anterior anchors to optimize the angles of tension for maximum effect.

In another aspect, the posterior anchor can include an expandable structure that can be collapsed so as to engage at least a portion of one side of the vessel in which it is deployed as well as to assume a reduced profile to allow improve blood flow therethrough. Example of such embodiments include a scaffold or wire form structure configured to be expanded within the vessel after delivery, then collapsed laterally by tensioning of the bridging element. Such embodiments can include a wire form structure having weakened portions extending longitudinally on opposite sides of the wire form structure to facilitate lateral collapse. The structures can be self-expanding or balloon deployable. In some embodiments, the collapsible wire form structure include one or more support ribs extending longitudinally to reinforce the collapsed structure to improve anchoring and adherence of the structure along a length of the body vessel. Such reinforcing ribs can be straight or can be curved as needed for a particular anatomy.

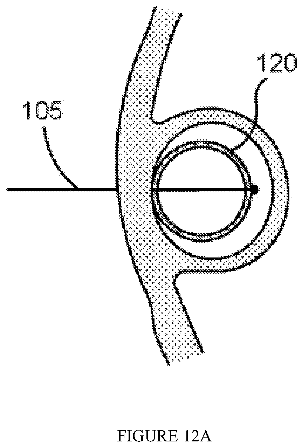

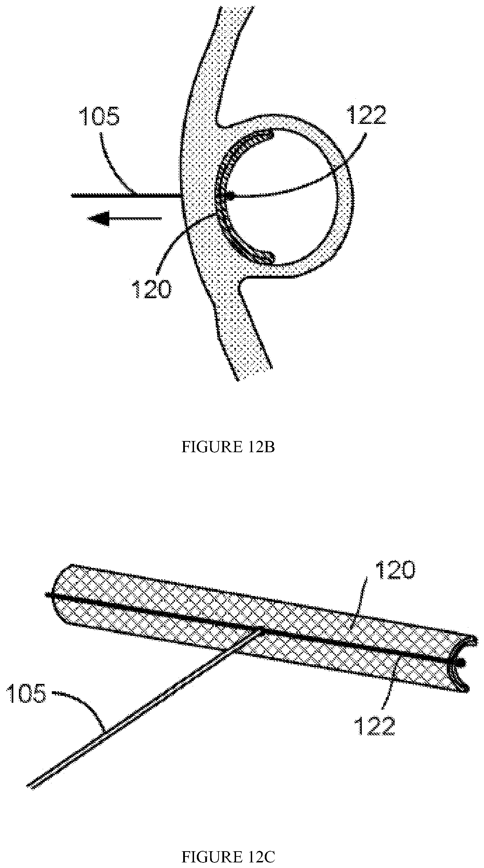

Figures 12A-12C and 13A-13B illustrate examples of the above described collapsible wire form cylinder structure 120. Typically, the wire form structure is a cylinder mesh structure that may be delivered in low profile and expand to the desired diameter, either by self-expansion or balloon expansion. The cylinder mesh structure can include a posterior backbone 122 that forms a T-bar and attaches to the bridging element 105.

As shown in Figure 12A, after deployment of the cylinder mesh structure 120 in a vessel, such as the GCV, the bridge element 105 extends to the support backbone 122 disposed on the opposing side of the cylindrical mesh structure 120 from where bridge element 105 extends through the wall of the GCV/LA. When tension is applied by the bridging element 105 to the backbone, the support crushes the cylinder mesh structure wall upon itself creating a flattened ribbon against the LA/GCV wall. Such a configuration is advantageous as it forms a stiff, relatively flat surface that effectively spreads the force of the tensioning against the wall to prevent the posterior anchor from being pulled through the GVC wall. Further, the folded design doubles the wall thickness and thus its strength and increases its purchase of the GCV wall up to 1.5 times its uncrushed diameter. Such a configuration allows for improved ease of deployment and allows the anchor to be embedded in the wall of the GVC upon deployment. Furthermore, the mesh structure of the scaffold further promotes tissue in-growth.

Figures 13A-13B illustrate another embodiment of a collapsible scaffold structure 120 that includes folding zones or softer sections 123 to insure preferential folding along predetermined lines. These folding zones extend longitudinally along most or all of the length of the cylindrical structure and can be defined by scores, weakened portions, or previous deformation to facilitate folding of the cylindrical mesh structure along these areas when deployed. Also, as with the crushable foam embodiment, the material or coating of the wire form structure, and the surface structure of the crushable wire form structure might be such that it spurs the ingrowth of tissue to, over time, form a tissue-anchor matrix. In either embodiment, the support backbone can be substantially straight, or preferably, curved to generally mimic the curve of the interior wall of the GVC. The scaffold can be a mesh structure, which can be defined to promote tissue-ingrowth.

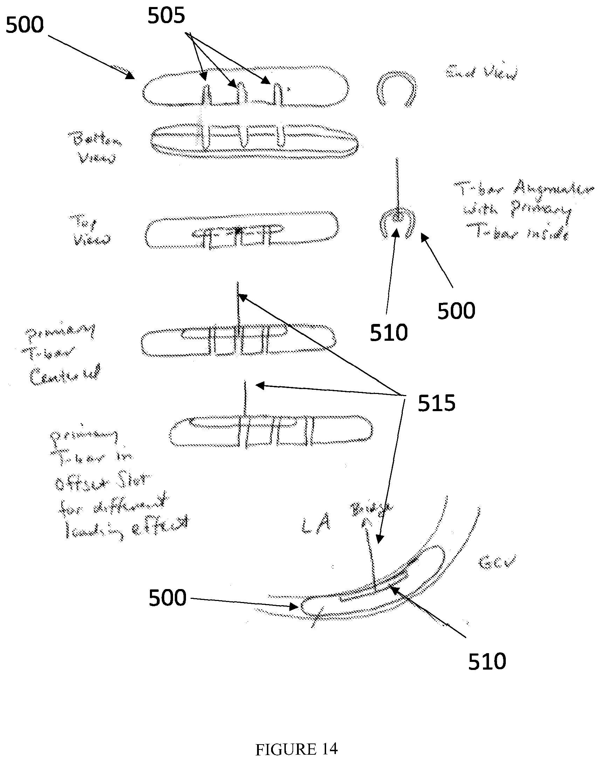

Figure 14 illustrates an augmentation device 500 which may be used with a posterior anchor of the invention that allows for variable loading effect on the atrial wall by the posterior anchor. As discussed herein, in various aspects, the invention provides a posterior anchor defined as a T-bar anchor 510 in which the bridging element 515 is attached to the center of the T-bar backbone. In some aspects, the augmentation device 500 is used with the T-bar anchor 510 of the invention and includes slot features 505 that allow the loading effect on the atrial wall by the T-bar anchor 510 to be varied in situ.

As such, the invention provides an anchor system that includes an augmentation device 500 of the invention and an anchor of the invention. In some aspects, the augmentation device 500 has an elongated cylindrical body defined by an elongated lumen having a substantially cylindrical wall. In some aspects, as shown in Figure 14, the lumen is configured to receive a T-bar anchor 510 and the cylindrical wall of the augmentation device includes slots 505 disposed along a length of the cylindrical body of the device for engaging a bridging element 515 of the anchor 510. In some aspects, the anchor 510 has a substantially cylindrical body that is sized to pass within the elongated cylindrical body of the augmentation device 500 and a bridging element 515 coupled to an intermediate portion of the anchor 510. It will be appreciated that the anchor for use with the augmentation device 500, may be any T-bar device disclosed herein or any other similarly shaped anchor device.

In practice, the augmentation device is delivered to the GCV to engage and augment a T-bar anchor of the invention once the T-bar anchor is delivered to the GVC. As shown in Figure 14, the augmentation device 500 is configured to be delivered in an orientation such that the device is parallel to the T-bar anchor 510 and then rotated so that the augmentation device 500 is sandwiched between the GCV inner wall and the T-bar anchor 510, with the bridge element 515 passing through one of the plurality of slots 505 on the augmentation device 500. The augmentation device allows compressive forces of the T-bar anchor to become spread more evenly over the GCV wall. Further, this spreading of forces can be varied and optimized by having different slots along the length of the augmentation device which the practitioner may choose to engage the bridging element.

It will be appreciated that the augmentation device 500 allows greater flexibility to the practitioner to modulate the outcome of the procedure intraprocedurally. Additionally, since the augmentation device provides a relatively larger area of contact with the GCV wall (compared to that of the T-bar anchor), a T-bar anchor of reduced length may be used making it easier to deliver and deploy.

It will also be appreciated that use of a T-bar anchor having a larger contact surface area in an effort to spread contact forces and reduce the potential for cutting and erosion of tissue has certain limitations. For example, it is typically difficult to deliver a wide or large T-bar anchor on the same catheter and at the same time a penetrating guidewire is being used to penetrate and cross the atrial wall during a procedure. The augmentation device 500 allows for the surgical step of crossing the atrial wall to be separated from the surgical step of deploying a relatively large T-bar anchor. Additionally, due to the multiple slots 505 on the augmentation device 500 that engage the bridging element 515, the effective attachment point of the bridge element 515 to the T-bar anchor 510 can be varied intraprocedurally.

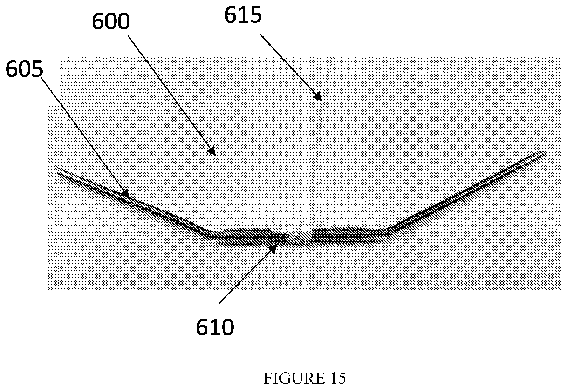

Figure 15 illustrates another configuration of an augmentation device 600 which may be used with a posterior anchor of one embodiment. As discussed herein, the augmentation device 600 is configured to add additional force vectors to the mechanism of shortening the A/P dimension of a mitral valve by allowing for asymmetric loading of the posterior anchor if necessary.

Further, the device 600 allows the practitioner to more specifically tailor the therapy to the patients anatomy by providing a supplemental implant variation that has different shapes, sizes or strengths. Accordingly,one embodiment provides and anchor system that includes an augmentation device 600 according to one embodiment and an anchor according to one embodiment, wherein the augmentation device is at least 1.5, 2, 3, 4, 5, 6, 7 or 8 times the length of the anchor device.

Figure 15 shows an anchor system which includes an augmentation device 600 having an elongated shaft body 605 composed of a shape memory material configured to deform from a first elongated configuration to a second flexed configuration. The second flexed configuration (shown in Figure 15) has a reduced length as compared to the first elongated configuration. The shaft body 605 is configured to conform to an anatomy of a patient in the second configuration upon deployment. The system further includes an anchor 610 having a substantially cylindrical body having a length less than the that of the augmentation device 600, and a bridging element 615 coupled to an intermediate portion of the anchor 610. In some aspects, the system is configured such that when the augmentation device 600 and anchor 610 are coupled upon deployment in a body lumen, a force upon a wall of the body lumen from the anchor 610 is translated to the augmentation device 600 to deform the wall.

As discussed herein, in certain aspects, a short T-bar anchor is utilized as the anchoring mechanism in the GCV to assist with delivery. Once the short T-bar anchor is positioned in the GCV, the augmentation device is positioned adjacent the T-bar anchor and coupled to the T-bar anchor. In some aspects, the augmentation device is composed of a shape memory material, such as nitinol wire, which allows the device to change from the first configuration to the second configuration. In another aspect, the augmentation device changes from the first configuration to the second configuration by a mechanical process, such as an adjustable linkage between portions of the device. The augmentation device is coupled to the T-bar anchor when deployed and applies a different application of force to the posterior wall as the anchor alone to reshape the annulus.

The present disclosure further provides devices and methods which allow for variable adjustment of the bridging element connecting one or more posterior anchors to one or more anterior anchors to reshape a body lumen, such as a heart chamber. As discussed herein, in certain aspects, the methods and devices are used to reshape the left atrium for treatment of a cardiac disease, such as mitral valve regurgitation. In various aspects, the devices and methods of the present disclosure provide a means in which the left atrium may be reshaped such that the regurgitation through the mitral valve is reduced or inhibited. It will be appreciated that this requires specific positioning of anchors and tensioning therebetween.

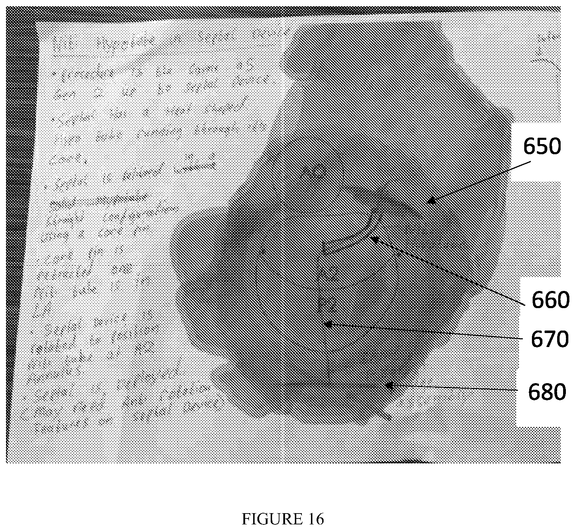

With reference to Figure 16, it is desirable to direct the force applied to the left atrium wall from the posterior anchor to a location close to A2 position of the atrium. This may be achieved in a number of ways as described herein. For example, in one aspect, the disclosure provides an anterior anchor 650 which is modified to include a tube 660 extending from the anchor into the atrial cavity to direct the force acting between the posterior anchor and anterior anchor towards an A2 position as shown in Figure 16. In this configuration, the tube 660 extends from the anchor 650 into the atrial cavity towards A2 such that the bridge element 670 connected to the posterior anchor 680 applies force in a more true AP orientation. During implantation, the anterior anchor 650 is delivered in a straight configuration using a core pin which is retracted once the tube 660 is in the left atrium. The anterior anchor 650 is then rotated to position the tube 660 at the A2 annulus and the anterior anchor is deployed and optionally includes anti-rotation features.

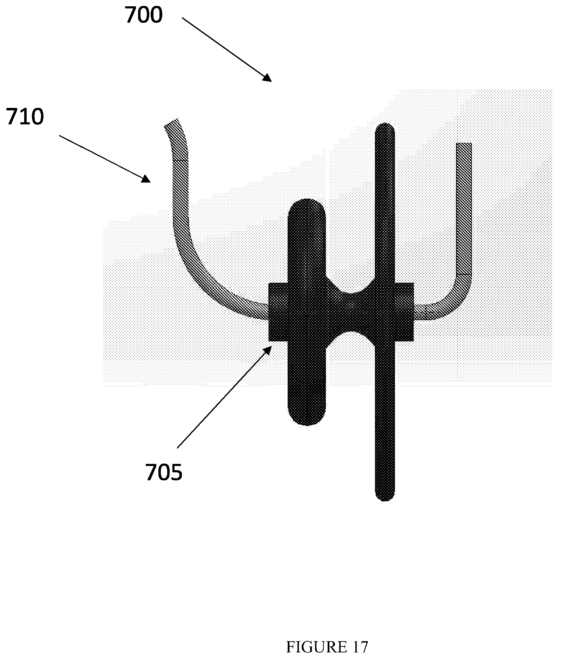

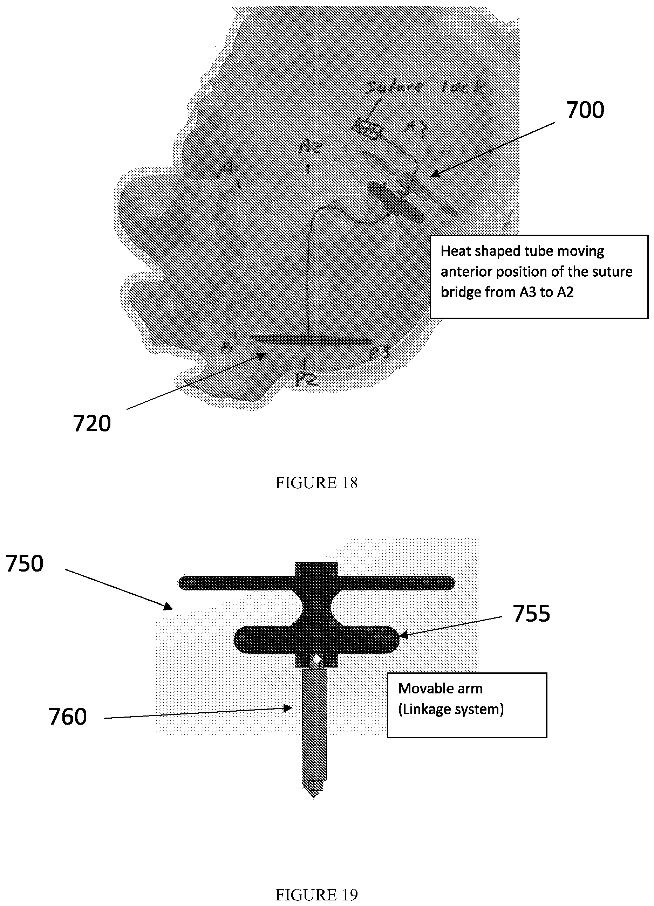

Figure 17 illustrates an anterior anchor 700 configured to locate the bridging element closer to an A2 location with the left atrium. The anchor 700 provides a means to reduce the AP dimension of the mitral valve using a posterior implant in the GCV and an anterior anchor 700 having a semi-rigid, pre-shaped hollow tube 710, e.g., "hypotube", running through it that repositions the bridging element location closer to A2. The anchor 700 allows for a more efficient AP diameter shortening by redirecting the bridging element to a trajectory that crosses the A2/P2 location of the mitral valve. The tube 710 extending from the body 705 of the anchor is used to direct the suture closer to A2. Figure 18 shows the anchor 700 of Figure 17 in a deployed configuration coupled to a posterior anchor 720 positioned at P2.

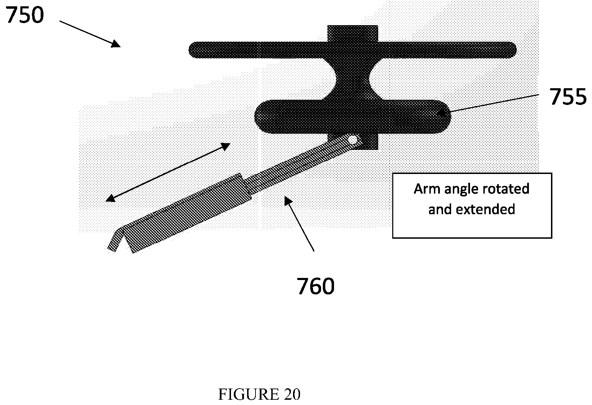

To achieve a similar outcome, the disclosure further provides an anterior anchor 750 having a movable arm 760 coupled to the body 755 of the anchor as shown in Figure 19. The adjustable arm mechanism is used to change the trajectory of the bridging element to provide a practitioner controlled movable linkage system. In practice, the anchor 750 is delivered over the crossing wire into the left atrium and the anterior anchor 750 is deployed as normal. In some aspects, the practitioner can adjust the anngle of the arm 760 as well as the length/extension of the arm to determine the best clinical result as shown in Figure 20. The arm angle and extension length of the arm are then locked in position.

For the aspects of the invention depicted in Figures 17, 19 and 20, the anchor is delivered in a straight configuration with the anchor collapsed. The anchor is loaded over the crossing wire/bridging element and deployed in the left atrium.

In some aspects, the anchor 700 shown in Figure 17 has a pre-shaped tubular rigid member which is used to keep the tube straight for insertion and then removed upon deployment to allow the tube 710 to take its heat shaped form thereby pushing the suture bridge anterior position closer to A2. In some aspects, the proximal portion of the pre-shaped tubing is shaped in a way to move the suture lock position as close to in-line with the trajectory of the bridging element crossing the mitral valve. This assists in balancing the moment created on the anchor by moving the crossing bridge away from the posterior anchor device center as shown in Figure 18.

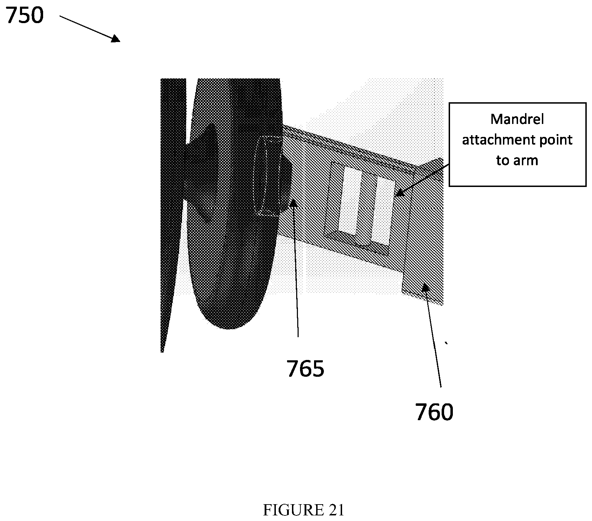

For the aspects of the disclosure depicted in Figures 19 and 20, once the anchor 750 has been deployed, 2 mandrels actuated by the practitioner at the proximal end of the deployment catheter are used to adjust the angle of rotation and the length of the extension of the arm 760. The angle is changed by pulling tension on the arm of the implant through a rotating hinge 765 as shown in Figure 21. In some aspects, the arm extension is performed by using an additional mandrel to push the arm 760 out along a track between the extension arm and the rotating arm as shown in Figure 20. Once the desired position has been determined both controls can be locked in place by locking the mandrels in position against the proximal side of the anchor. The mandrels are then disconnected proximal to the locking feature and the anchor 750 is deployed permentantly.

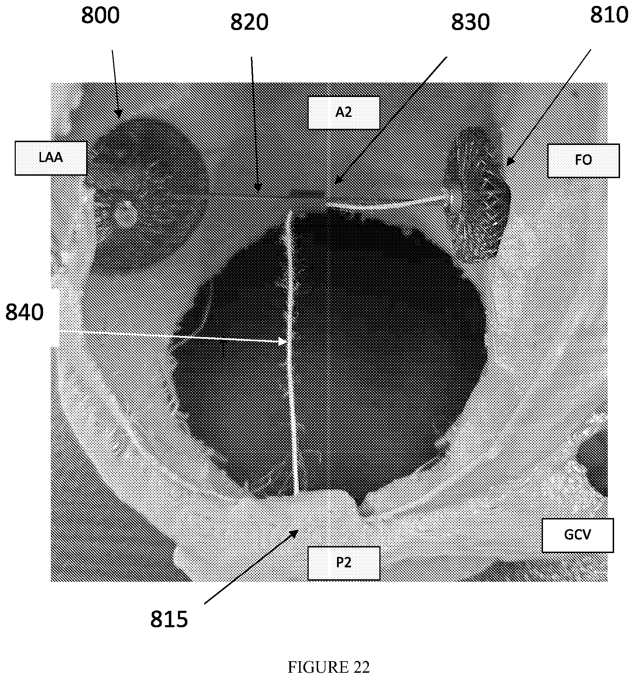

Figure 22 illustrates additional aspects of the disclosure which provide a means to reduce the AP dimension of the mitral valve using a posterior implant in the GCV and an anterior implant (including 2 connected anterior anchors, 800 and 810) that spans from the left atrial appendage (LAA) to the fossa ovalus (FO). As shown in Figure 22, the disclosure provides an implant system having a connecting rail 820 with a sliding lock 830 between anterior anchors 800 and 810 of the LAA and FO, the connecting rail 820 being coupled to the posterior anchor 815 via the suture bridge 840. This sliding lock can be positioned across the span from LAA to FO to achieve the most effective reduction of mitral valve regurgitation based on anatomy and disease state.

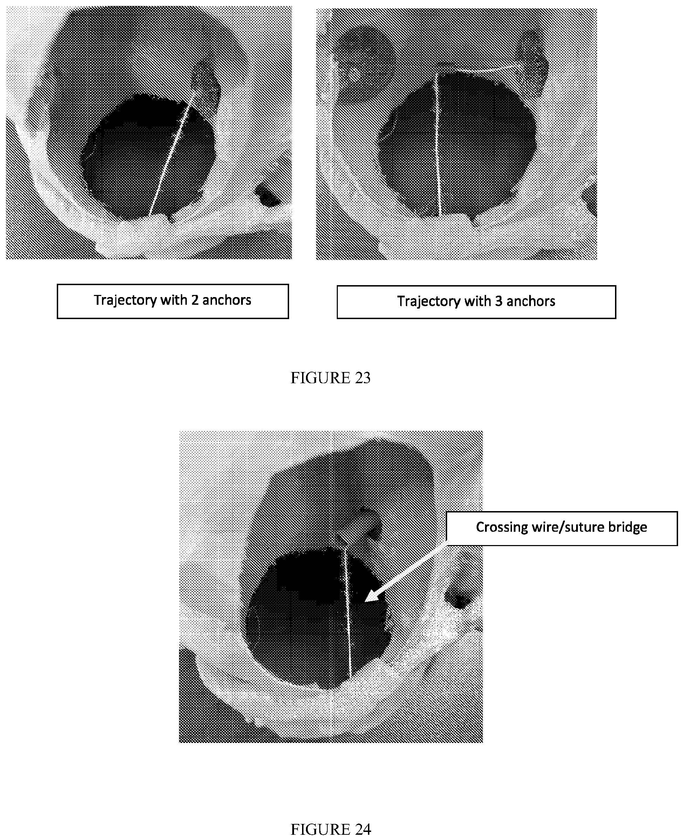

The aspect of the disclosure shown in Figure 22 allows the practitioner to pull the posterior anchor, e.g., T-bar anchor, from a location closer to A2 as discussed herein. This provides a more efficient AP shortening. It also allows the practitioner to tailor the therapy specific to where the regurgitant jet is present on the valve. Figure 23 illustrates the differences between an implant system having 3 anchors as opposed to 2 anchors.

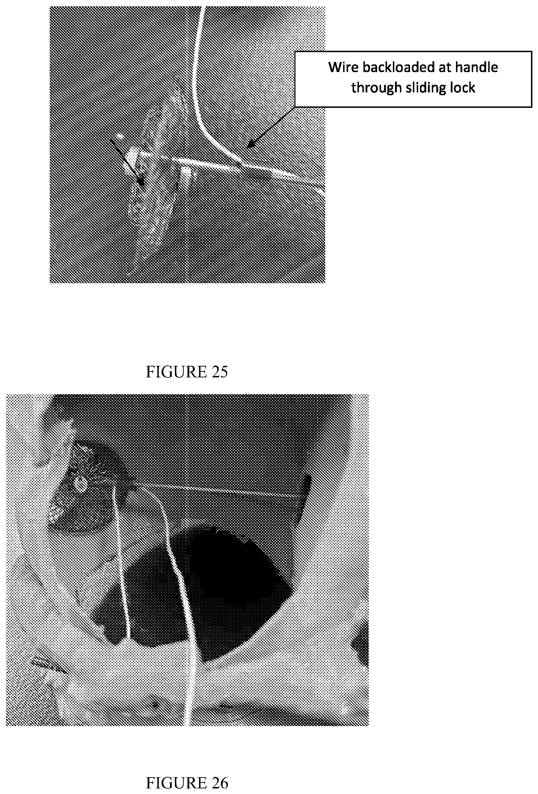

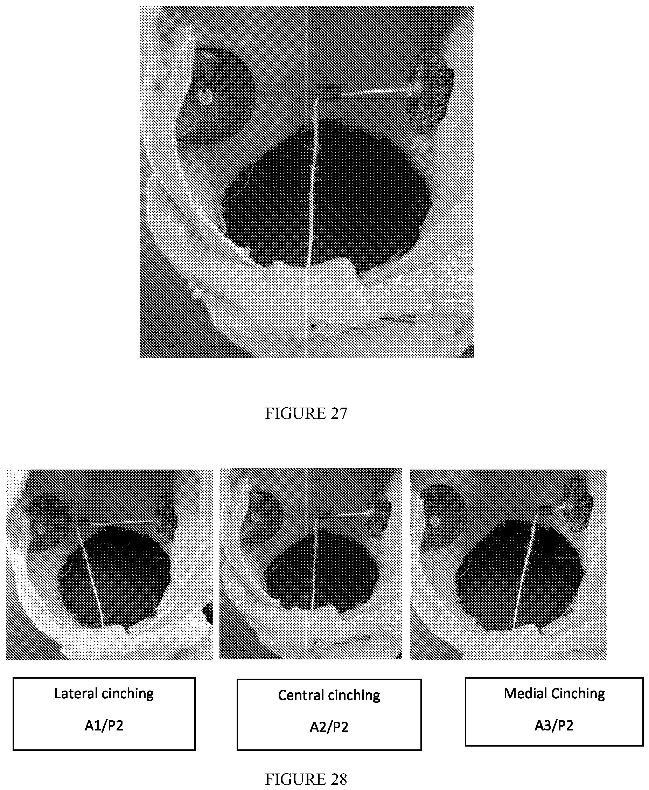

With reference to Figures 22-28, in practice, the implantation procedure proceeds as normal up until the wire crossing of the atrial wall is achieved and the left atrial catheter has been removed from the sheath. At this point a posterior anchor positioned in the GCV including a coupled bridging element crosses through the atrial wall, into the catheter (light blue) and out to the proximal end of the device as shown in Figure 24.

Over the crossing wire/bridging element, the anterior implant is then loaded. The anterior implant includes a first distal anterior anchor (displayed in the Figures as a nitinol wire vascular plug), a connecting rail (displayed as nitinol hypotube), a bridging element connector (displayed as a sliding lock) and a second distal anterior anchor. The first and second distal anterior anchors are connected by the connected rail. The cossing wire at the proximal end is backloaded into the sliding lock and through the distal implant grommet and the implant is advanced into the left atrium through the sheath.

The first distal anterior anchor, e.g., LAA anchor is advanced into the LAA and deployed there. In one aspect of the disclosure, the sheath is steerable to allow for wire crossing at P2 and to facilitate deployment in the LAA. The second distal anterior anchor, e.g., septal anchor, is then advanced and deployed in the septum. It is envisioned that the the anterior implant is delivered as a single implant (LAA anchor, sliding lock, rail and septal anchor) or discreet componts that are delivered sequentially. In some aspects, the LAA anchor is a nitinol mesh that expands into the LAA or an anchor type device that deploys into cardiac tissue or the fibrous skeleton of the heart, e.g., the left fibrous trigone. It will be appreciated that in some aspects, the delivery catheter is steerable to allow the first distal anterior anchor and the second distal anterior anchor to be delivered in the same catheter.

In some aspects, the septal implant is then deployed septally and the bridging element runs through the septal anchor, e.g., the second anterior anchor. The practitioner then begins to apply the therapy using echo doppler to assess the effectivness of the syncing. The disclosure provides two controls: 1) the practitioner can apply tension to the suture bridge to reduce the ap dimension; and/or 2) the practitioner can adjust the location of the sliding lock to change the angle of which the posterior anchor is being pulled.

Once the therapy has been applied, the sliding lock is locked in position on the sliding rail using a locking system similar to the suture lock. The suture will be locked on the right atrial side of the distal implant, e.g., second anterior anchor. The procedure then continues such that the suture lock is deployed and the suture is cut.

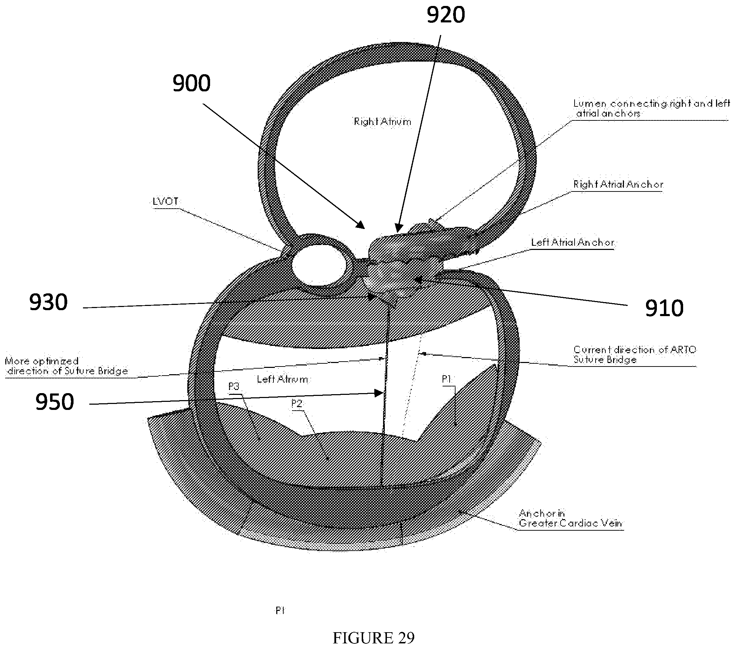

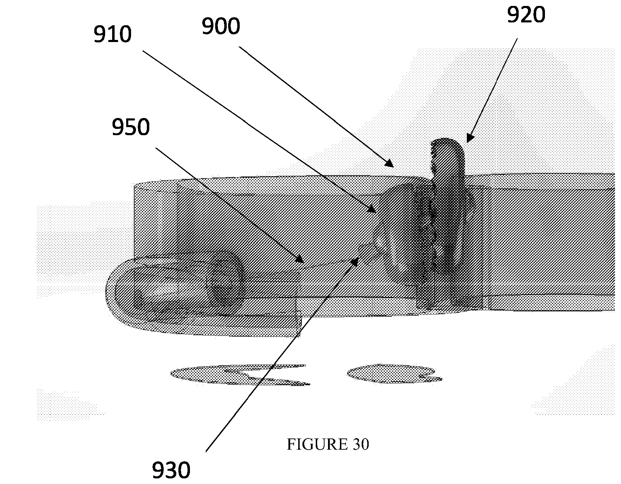

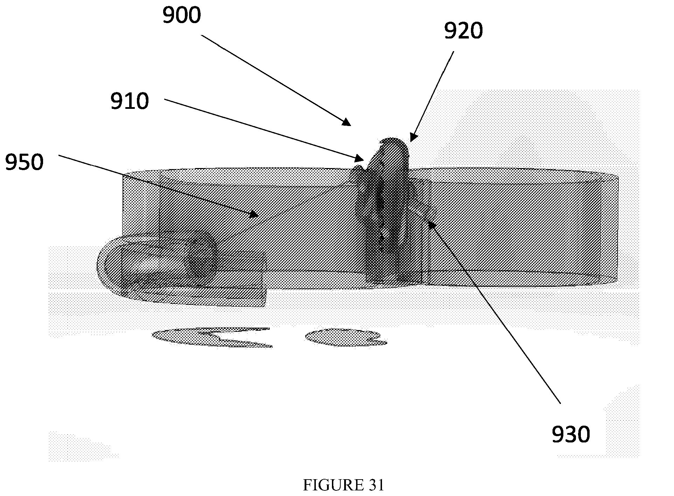

Figures 29-31 illustrate an anterior anchor 900 configured to locate the bridging element 950 closer to an A2 location with the left atrium in another aspect of the disclosure. To alter the direction of tensioning between the puncture site of the septal wall and the puncture site of the GCV, the anterior anchor 900 includes a left anchor member 910 and a right anchor member 920 which are placed independent of one another. This changes the angle and direction of the bridging element 950 coupled to the posterior anchor and provides a more directed therapy for the patient.

Unlike a convention anterior anchor having a coaxial through hole, the anchor 900 shown in Figures 29-31 provides a dual anterior anchor in which the left anchor member 910 and the right anchor member 920 are positioned separately. Through holes of each member are connected by a tubular lumen 930. As shown in the Figures, the through holes are not coaxially aligned thereby directing the bridging element 950, e.g., suture bridge, to apply the most effective tensioning therapy.

The foregoing is considered as illustrative only of the principles of the invention. The embodiments herein disclosed merely exemplify the invention which may be embodied in other specific structures. While preferred embodiments have been described, the details may be changed without departing from the invention. Further, most of the inventions are shown in simple forms to illustrate elemental function and features and may be combined to a final embodiment that uses one more elements combined into a single device. It is also anticipated that the embodiments described may be combined, by way of example but not by way of limitation, having a curbed backbone in the crushable foam, or multiple curved anchors with anti-flipping features or configurations with multiple attachments to the anterior anchor. Furthermore, since numerous modifications and changes will readily occur to those skilled in the art, the invention is not limited to the construction and operation shown and described in the preferred embodiments except as limited by the claims.

Although the invention has been described with reference to the above examples, it will be understood that modifications and variations are encompassed within the scope of the invention. Accordingly, the invention is limited only by the following claims.

## Claims

1. An anchor system for reshaping a heart valve annulus for treatment of a heart disorder, the anchor system comprising:
a) an augmentation device (500) having an elongated cylindrical body, the augmentation device (500) having an elongated lumen defined by a substantially cylindrical wall, the lumen being configured to receive an anchor (510), wherein the cylindrical wall includes a plurality of slots (505) disposed along a length of the cylindrical body for engaging a bridging element (515) of the anchor (510) ; and
b) an anchor (510) having a substantially cylindrical body that is sized to pass within the elongated cylindrical body of the augmentation device (500) and a bridging element (515) coupled to an intermediate portion of the cylindrical body of the anchor.

2. The anchor system of claim 1, wherein the anchor (510) further comprises a substantially rigid backbone extending longitudinally along at least a portion of the cylindrical body of the anchor (510); optionally wherein the substantially rigid backbone is disposed on the cylindrical body of the anchor (510).

3. The anchor system of claim 1, wherein the augmentation device (500) and the anchor (510) are longitudinally curved so as to conform to anatomy of a patient.

4. The anchor system of claim 1, wherein the augmentation device (500) comprises at least 3, 4, 5, 6, 7, 8, 9, 10 or more slots (505) disposed along its length.

## Patentansprüche

1. Ankersystem zum Umformen eines Herzklappenanulus zur Behandlung einer Herzerkrankung, wobei das Ankersystem umfasst:
a) eine Erweiterungsvorrichtung (500) mit einem langgestreckten zylindrischen Körper, wobei die Erweiterungsvorrichtung (500) ein langgestrecktes Lumen aufweist, das durch eine im Wesentlichen zylindrische Wand definiert ist, wobei das Lumen zum Aufnehmen eines Ankers (510) gestaltet ist, wobei die zylindrische Wand eine Vielzahl von Schlitzen (505) aufweist, die entlang einer Länge des zylindrischen Körpers zum Eingriff mit einem Verbrückungselement (515) des Ankers (510) angeordnet sind; und
b) einen Anker (510) mit einem im Wesentlichen zylindrischen Körper, der bemessen ist, innerhalb des langgestreckten zylindrischen Körpers der Erweiterungsvorrichtung (500) zu verlaufen, und einem Verbrückungselement (515), das an einen Zwischenabschnitt des zylindrischen Körpers des Ankers gekoppelt ist.

2. Ankersystem nach Anspruch 1, wobei der Anker (510) ferner ein im Wesentlichen starres Gerüst umfasst, das in Längsrichtung entlang wenigstens eines Abschnitts des zylindrischen Körpers des Ankers (510) verläuft; wobei gegebenenfalls das im Wesentlichen starre Gerüst an dem zylindrischen Körper des Ankers (510) angeordnet ist.

3. Ankersystem nach Anspruch 1, wobei die Erweiterungsvorrichtung (500) und der Anker (510) in Längsrichtung gekrümmt sind, um sich der Anatomie eines Patienten anzupassen.

4. Ankersystem nach Anspruch 1, wobei die Erweiterungsvorrichtung (500) wenigstens 3, 4, 5, 6, 7, 8, 9, 10 oder mehr Schlitze (505) umfasst, die entlang ihrer Länge angeordnet sind.

## Revendications

1. Système d'ancrage pour le remodelage d'un anneau de valve cardiaque pour le traitement d'un trouble cardiaque, le système d'ancrage comprenant :
a) un dispositif d'augmentation (500) ayant un corps cylindrique allongé, le dispositif d'augmentation (500) ayant une lumière allongée définie par une paroi sensiblement cylindrique, la lumière étant configurée pour recevoir un ancrage (510), la paroi cylindrique comprenant une pluralité de fentes (505) disposées sur une longueur du corps cylindrique pour venir en prise avec un élément de pontage (515) de l'ancrage (510) ; et
b) un ancrage (510) ayant un corps sensiblement cylindrique dimensionné pour passer à l'intérieur du corps cylindrique allongé du dispositif d'augmentation (500) et un élément de pontage (515) couplé à une partie intermédiaire du corps cylindrique de l'ancrage.

2. Système d'ancrage selon la revendication 1, l'ancrage (510) comprenant en outre une ossature sensiblement rigide s'étendant longitudinalement le long d'au moins une partie du corps cylindrique de l'ancrage (510) ; éventuellement, l'ossature sensiblement rigide étant disposée sur le corps cylindrique de l'ancrage (510).

3. Système d'ancrage selon la revendication 1, le dispositif d'augmentation (500) et l'ancrage (510) étant courbés longitudinalement de façon à se conformer à l'anatomie d'un patient.

4. Système d'ancrage selon la revendication 1, le dispositif d'augmentation (500) comprenant au moins 3, 4, 5, 6, 7, 8, 9, 10 fentes ou plus (505) disposées sur sa longueur.
